(19) Europäisches Patentamt
European Patent Office
Office européen des brevets

(11) EP 3 641 723 B1

(12) **EUROPEAN PATENT SPECIFICATION**

(45) Date of publication and mention of the grant of the patent:
**09.11.2022 Bulletin 2022/45**

(21) Application number: **18733253.1**

(22) Date of filing: **21.06.2018**

(51) International Patent Classification (IPC):
***A61K 8/41*** *(2006.01)* ***A61Q 5/12*** *(2006.01)*
***A61K 8/891*** *(2006.01)* ***A61K 8/898*** *(2006.01)*

(52) Cooperative Patent Classification (CPC):
**A61K 8/416; A61K 8/891; A61K 8/898; A61Q 5/12**

(86) International application number:
**PCT/EP2018/066571**

(87) International publication number:
**WO 2018/234444 (27.12.2018 Gazette 2018/52)**

(54) **COSMETIC COMPOSITION COMPRISING TWO SPECIFIC CATIONIC SURFACTANTS AND A SILICONE EMULSION, AND A COSMETIC TREATMENT PROCESS**

KOSMETISCHE ZUSAMMENSETZUNG MIT ZWEI SPEZIFISCHEN KATIONISCHEN TENSIDEN UND SILIKONEMULSION UND KOSMETISCHES BEHANDLUNGSVERFAHREN

COMPOSITION COSMÉTIQUE COMPRENANT DEUX TENSIOACTIFS CATIONIQUES SPÉCIFIQUES ET UNE ÉMULSION DE SILICONE, ET PROCÉDÉ DE TRAITEMENT COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2017 FR 1755647**

(43) Date of publication of application:
**29.04.2020 Bulletin 2020/18**

(73) Proprietor: **L'Oreal**
**75008 Paris (FR)**

(72) Inventors:
- **LESAULNIER, Claire-Marie**
  **93400 Saint-Ouen (FR)**
- **CHESNEAU, Laurent**
  **93400 Saint-Ouen (FR)**

(74) Representative: **L'Oreal**
**Service D.I.P.I.**
**9, rue Pierre Dreyfus**
**92110 Clichy (FR)**

(56) References cited:
**EP-A1- 0 916 690**
**EP-A2- 1 627 625**
**JP-A- 2000 219 612**
**JP-A- 2001 139 429**
**JP-A- 2005 213 163**
**US-A1- 2010 015 078**

EP 3 641 723 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a cosmetic composition, in particular a hair composition, comprising the combination of at least two specific cationic surfactants and a silicone emulsion; and also to a cosmetic treatment process using this composition.

In the field of the cosmetic treatment of keratin fibres, in particular human keratin fibres, and in particular the hair, it is sought to treat keratin fibres that have been sensitized and/or damaged, in particular following various external attacks, such as mechanical attacks, for example linked to disentangling or blow drying, or else chemical attacks, for example linked to dyeing or permanent-waving.

These attacks can have repercussions on the quality of keratin fibres, and can in particular cause a degradation of the surface properties of keratin fibres, which become unsmooth and uneven at the surface, hence they are not as easy to disentangle and have a less pleasant feel, whether on dry hair or wet hair.

Care products exist which make it possible to limit these phenomena. These products generally contain one or more conditioning agents, such as cationic surfactants, fatty alcohols or silicones. Examples of such products are described in JP 2005 213163 A, JP 2000 219612 A, JP 2001 139429 A and US 2010/015078 A1.

However, some of these conditioning agents have the drawback of thickening the texture of hair care products and of resulting in an excessively high viscosity of the compositions; some conditioning agents also have the drawback of depositing too much conditioning agent on the hair, which generally results in a softening or a lack of tonicity of the fibre, and/or a lack of resistance of the fibre, and also in a feeling of being unclean and greasy to the touch, due in particular to excessively rapid regreasing between two shampooing operations.

The applicant has now discovered that the combination of at least two specific cationic surfactants and of an emulsion of at least two silicones makes it possible to overcome the drawbacks set out above.

One subject of the invention is thus a cosmetic composition, in particular a hair composition, comprising:

(i) one or more cationic surfactants of formula (Ia):

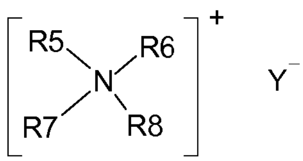

(Ia)

in which:

- R5 and R6, which may be identical or different, represent a linear or branched, saturated or unsaturated aliphatic group comprising from 8 to 30 carbon atoms, preferably from 12 to 24 carbon atoms, said aliphatic group possibly comprising one or more heteroatoms such as oxygen, nitrogen, sulfur and/or halogens; or an aromatic group such as an aryl or alkaryl group, in particular a benzyl group;
- R7 and R8, which may be identical or different, represent a linear or branched aliphatic group comprising from 1 to 6 carbon atoms;
- $Y^-$ is an anion;

(ii) one or more cationic surfactants other than the cationic surfactants of formula (Ia), and preferably corresponding to formula (Ib) below:

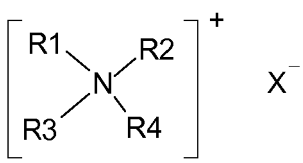

(Ib)

in which:

- R1 represents a linear or branched, saturated or unsaturated aliphatic group comprising from 8 to 30 carbon atoms, preferably from 12 to 24 carbon atoms, said aliphatic group possibly comprising one or more heteroatoms such as oxygen, nitrogen, sulfur and/or halogens;
- R2 to R4, which may be identical or different, represent a linear or branched, saturated or unsaturated aliphatic group comprising from 1 to 6 carbon atoms;

- $X^-$ is an anion; and

(iii) an oil-in-water emulsion having a particle size D50 of less than 350 nm, and comprising:

- a silicone mixture comprising (i) a polydialkylsiloxane comprising trialkylsilyl end groups, having a viscosity at 25°C ranging from 40 000 to 100 000 mPa.s and (ii) an aminosilicone having a viscosity at 25°C ranging from 1000 to 15 000 mPa.s and an amine number ranging from 2 to 10 mg of KOH per gram of aminosilicone;
- a surfactant mixture comprising one or more non-ionic surfactants, said mixture having an HLB ranging from 10 to 16, and
- water.

The composition according to the invention has good cosmetic properties, in particular conditioning cosmetic properties; it in particular makes it possible to obtain high performance levels in terms of visual smoothness and smoothness to the touch (smooth nature) of the hair, said smoothness being noticeable over the entire hair, that is to say from the root of the hair to the end thereof (root-end smoothness).
In addition, the composition according to the invention gives the keratin fibres tonicity.
It also makes it possible to obtain a non-lasting "cleanness" effect, that is to say that the hair has a non-greasy, natural feel which endures between two shampooing operations.
The composition according to the invention also makes it possible to deposit the required amount of conditioning agents to obtain very good performance levels, in particular in terms of smoothness, while at the same time avoiding excessive deposition which would be detrimental to the tonicity of the fibres and their clean feel.
It is particularly suitable for sensitized hair, in particular damaged, split, brittle hair, to which it can provide strength, vitality and body, said hair thus appearing revitalized, repaired and reinforced.
The composition according to the invention has, moreover, a viscosity suitable for easy application, without risk of it running. It has been noted that this viscosity changes little after a prolonged storage period.
Advantageously, the composition according to the invention has a viscosity of greater than 800 centipoises (cps), preferably greater than 900 cps, better still of between 900 and 1500 cps (viscosity measured using a Rheomat RM 200, at 25°C, spindle 3, 200 revolutions/min, measured at 30 s).
In the text hereinbelow, and unless otherwise indicated, the limits of a range of values are included in that range, in particular in the expressions "*between*" and "*ranging from ... to ...*".
The expression "*at least one*" used in the present description is equivalent to the expression "*one or more*".

<u>(i) Cationic surfactants of formula (Ia)</u>

The composition according to the invention comprises one or more surfactants of formula (Ia) as defined above.
Preferably, R5 represents a benzyl group or a linear or branched, saturated aliphatic group comprising from 8 to 30 carbon atoms, preferably from 12 to 24 carbon atoms, said aliphatic group possibly comprising one or more heteroatoms such as oxygen and/or nitrogen.
Preferably, R6 represents a benzyl group or a linear or branched, saturated aliphatic group comprising from 8 to 30 carbon atoms, preferably from 12 to 24 carbon atoms, said aliphatic group possibly comprising one or more heteroatoms such as oxygen and/or nitrogen.
The aliphatic groups are for example chosen from C8-C30 alkyl, C8-C30 alkoxy, (C2-C6)polyoxyalkylene, C8-C30 alkylamide, (C12-C22)alkylamido(C2-C6)alkyl, (C12-C22)alkyl acetate and C8-C30 hydroxyalkyl groups.
In particular, R5 may be a saturated linear C8-C30, in particular C12-C24, alkyl group, or a (C12-C22)alkylamido(C2-C6)alkyl group. Preferentially, R5 may be a behenyl, cetyl, stearyl or stearamidopropyl radical.
In particular, R6 may be a saturated linear C8-C30, in particular C12-C24, alkyl group, or a (C12-C22)alkyl acetate group. Preferentially, R6 may be a benzyl, behenyl, cetyl, stearyl or myristyl acetate radical.
Preferably, R7 and R8, which may be identical or different, represent a saturated linear alkyl group comprising from 1 to 6 carbon atoms, in particular from 1 to 3 carbon atoms.
Preferentially, R7 and R8 represent a methyl group.
Preferably, $Y^-$ is an anion chosen from halides such as chloride, bromide and iodide; phosphates, acetates, lactates, (C1-C4)alkyl sulfates, (C1-C4)alkyl- or (C1-C4)alkylarylsulfonates.
Preferably, the cationic surfactant(s) of formula (Ia) are chosen from di(C8-C30)alkyldimethylammonium salts, and in particular distearyldimethylammonium salts, dicetyldimethylammonium salts, benzyldimethylstearylammonium salts, stearamidopropyldimethyl(myristyl acetate)ammonium salts, and mixtures thereof. More particularly, they are chosen from di(C8-C30)alkyldimethylammonium halides, in particular chlorides, and in particular distearyldimethylammonium halides, in particular chlorides, dicetyldimethylammonium halides, in particular chlorides, benzyldimethylstearylammonium halides, in particular chlorides, stearamidopropyldimethyl(myristyl acetate)ammonium halides, in particular

chlorides; or else from di(C8-C30)alkyldimethylammonium (C1-C4)alkyl sulfates, in particular methosulfates, and in particular distearyldimethylammonium (C1-C4)alkyl sulfates, in particular methosulfates, dicetyldimethylammonium(C1-C4)alkyl sulfates, in particular methosulfates, benzyldimethylstearylammonium (C1-C4)alkyl sulfates, in particular methosulfates, stearamidopropyldimethyl(myristyl acetate)ammonium (C1-C4)alkyl sulfates, in particular methosulfates; and also mixtures thereof.

Preferentially, the cationic surfactant(s) of formula (Ia) are chosen from dicetyldimethylammonium halides, in particular chlorides, or dicetyldimethylammonium (C1-C4)alkyl sulfates, in particular methosulfates.

The composition according to the invention preferably comprises the surfactant(s) of formula (Ia) in a total amount ranging from 0.1% to 10% by weight, better still from 0.2% to 10% by weight, preferentially from 0.5% to 5% by weight, relative to the total weight of the composition.

(ii) Additional cationic surfactants

The composition according to the also invention comprises one or more cationic surfactants different from the cationic surfactants of formula (Ia) as defined above.

The term "cationic surfactant" is intended to mean a surfactant that is positively charged when it is contained in the composition according to the invention. This surfactant may bear one or more positive permanent charges or may contain one or more cationizable functions in the composition according to the invention.

The additional cationic surfactant(s) (ii) are preferably chosen from primary, secondary or tertiary fatty amines, which are optionally polyoxyalkylenated, or salts thereof, and quaternary ammonium salts, and mixtures thereof.

The fatty amines generally comprise at least one $C_8$ to $C_{30}$ hydrocarbon-based chain.

Among the quaternary ammonium salts, mention may be made in particular of:

(a) the cationic surfactants of formula (Ib):

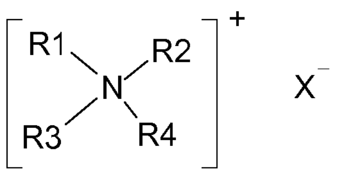

(Ib)

in which:

- R1 represents a linear or branched, saturated or unsaturated aliphatic group comprising from 8 to 30 carbon atoms, preferably from 12 to 24 carbon atoms, said aliphatic group possibly comprising one or more heteroatoms such as oxygen, nitrogen, sulfur and/or halogens;
- R2 to R4, which may be identical or different, represent a linear or branched, saturated or unsaturated aliphatic group comprising from 1 to 6 carbon atoms;
- $X^-$ is an anion.

Preferably, R1 represents a linear or branched, saturated aliphatic group comprising from 8 to 30 carbon atoms, preferably from 12 to 24 carbon atoms, said aliphatic group possibly comprising one or more heteroatoms such as oxygen and/or nitrogen.

The aliphatic groups are for example chosen from C8-C30 alkyl, C8-C30 alkoxy, (C2-C6)polyoxyalkylene, C8-C30 alkylamide, (C12-C22)alkylamido(C2-C6)alkyl, (C12-C22)alkyl acetate and C8-C30 hydroxyalkyl groups.

In particular, R1 may be a saturated linear C8-C30, in particular C12-C24, alkyl group, or a (C12-C22)alkylamido(C2-C6)alkyl group. Preferentially, R1 may be a behenyl, cetyl or palmitylamidopropyl radical.

Preferably, R2 to R4, which may be identical or different, represent a saturated linear alkyl group comprising from 1 to 6 carbon atoms, in particular from 1 to 3 carbon atoms. Preferentially, R2, R3 and R4 represent a methyl group.

Preferably, $X^-$ is an anion chosen from halides such as chloride, bromide and iodide; phosphates, acetates, lactates, (C1-C4)alkyl sulfates, (C1-C4)alkyl- or (C1-C4)alkylarylsulfonates.

(b) quaternary ammonium salts of imidazoline, for instance those of formula (II) below:

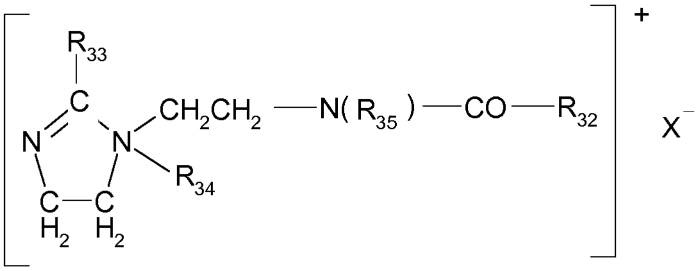

in which:

- $R_{32}$ represents an alkenyl or alkyl group comprising from 8 to 30 carbon atoms, for example derived from tallow fatty acids,
- $R_{33}$ represents a hydrogen atom, a $C_1$ to $C_4$ alkyl group or an alkenyl or alkyl group comprising from 8 to 30 carbon atoms,
- $R_{34}$ represents a $C_1$ to $C_4$ alkyl group,
- $R_{35}$ represents a hydrogen atom or a $C_1$ to $C_4$ alkyl group, and
- $X^-$ is an anion, in particular chosen from the group of halides, phosphates, acetates, lactates, alkyl sulfates, alkyl- or alkylarylsulfonates in which the alkyl and aryl groups preferably comprise, respectively, from 1 to 20 carbon atoms and from 6 to 30 carbon atoms.

Preferably, $R_{32}$ and $R_{33}$ denote a mixture of alkenyl or alkyl groups comprising from 12 to 21 carbon atoms, for example derived from tallow fatty acids, $R_{34}$ denotes a methyl group and $R_{35}$ denotes a hydrogen atom. Such a product is sold, for example, under the name Rewoquat® W 75 by Rewo;
(c) di- or triquaternary ammonium salts, in particular of formula (III):

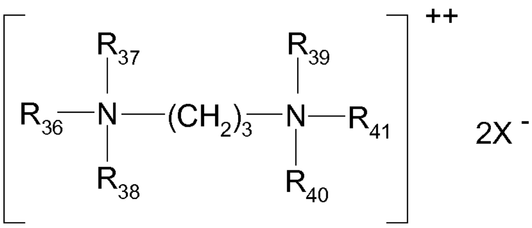

in which

- $R_{36}$ denotes an alkyl radical comprising approximately from 16 to 30 carbon atoms, which is optionally hydroxylated and/or interrupted with one or more oxygen atoms,
- $R_{37}$ is chosen from hydrogen or an alkyl radical comprising from 1 to 4 carbon atoms or a (R36a)(R37a)(R38a)N-(CH2)3 group, $R_{36a}$, $R_{37a}$, $R_{38a}$, $R_{38}$, $R_{39}$, $R_{40}$ and $R_{41}$, which may be identical or different, are chosen from hydrogen or an alkyl group comprising from 1 to 4 carbon atoms, and
- $X^-$ is an anion, in particular chosen from the group of halides, acetates, phosphates, nitrates and methyl sulfates.

Such compounds are, for example, Finquat CT-P, sold by the company Finetex (Quaternium 89), and Finquat CT, sold by the company Finetex (Quaternium 75), (d) quaternary ammonium salts containing at least one ester function, such as those having formula (IV) below:

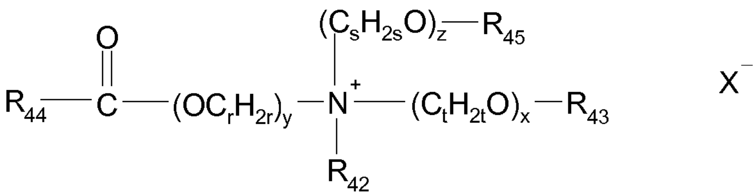

in which:

- $R_{42}$ is chosen from $C_1$ to $C_6$ alkyl groups and $C_1$ to $C_6$ hydroxyalkyl ordihydroxyalkyl groups;
- $R_{43}$ is chosen from:

  - the group

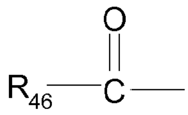

  - groups $R_{47}$, which are linear or branched, saturated or unsaturated $C_1$ to $C_{22}$ hydrocarbon-based groups,
  - a hydrogen atom,

  $R_{45}$ is chosen from:

  - the group

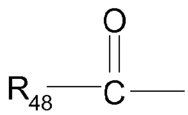

  - groups $R_{49}$, which are linear or branched, saturated or unsaturated $C_1$ to $C_6$ hydrocarbon-based groups,
  - a hydrogen atom,

- $R_{44}$, $R_{46}$ and $R_{48}$, which may be identical or different, are chosen from linear or branched, saturated or unsaturated $C_7$ to $C_{21}$ hydrocarbon-based groups;
- r, s and t, which may be identical or different, are integers ranging from 2 to 6;
- y is an integer ranging from 1 to 10;
- x and z, which may be identical or different, are integers ranging from 0 to 10;
- $X^-$ is a simple or complex and organic or mineral anion;

with the proviso that the sum x + y + z is from 1 to 15, that when x is 0 then $R_{43}$ denotes $R_{47}$, and that when z is 0 then $R_{45}$ denotes $R_{49}$.

The alkyl groups $R_{42}$ may be linear or branched, and more particularly linear. Preferably, $R_{42}$ denotes a methyl, ethyl, hydroxyethyl or dihydroxypropyl group, and more particularly a methyl or ethyl group.

Advantageously, the sum x + y + z is from 1 to 10.

When $R_{43}$ is a hydrocarbon-based group $R_{47}$, it may comprise from 12 to 22 carbon atoms, or else from 1 to 3 carbon atoms.

When $R_{45}$ is a hydrocarbon-based group $R_{49}$, it preferably contains 1 to 3 carbon atoms.

Advantageously, $R_{44}$, $R_{46}$ and $R_{48}$, which may be identical or different, are chosen from linear or branched, saturated or unsaturated $C_{11}$ to $C_{21}$ hydrocarbon-based groups, and more particularly from linear or branched, saturated or unsaturated $C_{11}$ to $C_{21}$ alkyl and alkenyl groups.

Preferably, x and z, which may be identical or different, are equal to 0 or 1. Advantageously, y is equal to 1.

Preferably, r, s and t, which may be identical or different, are equal to 2 or 3, and even more particularly are equal to 2.

The anion $X^-$ is preferably a halide (chloride, bromide or iodide) or an alkyl sulfate, more particularly methyl sulfate. However, use may be made of methanesulfonate, phosphate, nitrate, tosylate, an anion derived from an organic acid, such as acetate or lactate, or any other anion that is compatible with the ammonium bearing an ester function.

The anion $X^-$ is even more particularly chloride or methyl sulfate.

Use is more particularly made, in the composition according to the invention, of the ammonium salts of formula (IV) in which:

$R_{42}$ denotes a methyl or ethyl group,
x and y are equal to 1;
z is equal to 0 or 1;
r, s and t are equal to 2;
$R_{43}$ is chosen from:

- the group

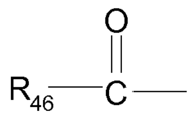

- methyl, ethyl or $C_{14}$ to $C_{22}$ hydrocarbon-based groups,
- a hydrogen atom;

$R_{45}$ is chosen from:

- the group

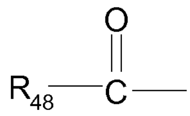

- a hydrogen atom;

$R_{44}$, $R_{46}$ and $R_{48}$, which may be identical or different, are chosen from saturated or unsaturated, linear or branched $C_{13}$ to $C_{17}$ hydrocarbon-based groups and preferably from saturated or unsaturated, linear or branched $C_{13}$ to $C_{17}$ alkyl and alkenyl groups.

Advantageously, the hydrocarbon-based groups are linear.

Mention may for example be made of the salts (chloride or methyl sulfate in particular) of diacyloxyethyldimethylammonium, of diacyloxyethylhydroxyethyldimethylammonium, of monoacyloxyethylhydroxyethyldimethylammonium, of triacyloxyethylmethylammonium, of monoacyloxyethylhydroxyethyldimethylammonium, and mixtures thereof. The acyl groups preferably contain 14 to 18 carbon atoms and are derived more particularly from a plant oil such as palm oil or sunflower oil. When the compound contains several acyl groups, these groups may be identical or different.

These products are obtained, for example, by direct esterification of triethanolamine, triisopropanolamine, alkyldiethanolamine or alkyldiisopropanolamine, which are optionally oxyalkylenated, with $C_{10}$ to $C_{30}$ fatty acids or with mixtures of $C_{10}$ to $C_{30}$ fatty acids of plant or animal origin, or by transesterification of their methyl esters. This esterification is followed by a quaternization using an alkylating agent such as an alkyl halide (preferably a methyl or ethyl halide), a dialkyl sulfate (preferably a methyl or ethyl sulfate), methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company CECA or Rewoquat® WE 18 by the company Rewo-Witco.

Preferably, the composition according the invention comprises one or more cationic surfactants of formula (Ib), and more particularly one or more cationic surfactants chosen from (C8-C30)alkyltrimethylammonium salts, and in particular cetyltrimethylammonium salts, behenyltrimethylammonium salts, palmitylamidopropyltrimethylammonium salts, and mixtures thereof.

More particularly, the composition comprises one or more cationic surfactants chosen from (C8-C30)alkyltrimethylammonium halides, in particular chlorides, cetyltrimethylammonium halides, in particular chloride, behenyltrimethylammonium halides, in particular chloride, palmitylamidopropyltrimethylammonium halides, in particular chloride; or (C8-C30)alkyltrimethylammonium (C1-C4)alkyl sulfates, in particular methosulfates, and in particular cetyltrimethylammonium (C1-C4)alkyl sulfates, in particular methosulfate, behenyltrimethylammonium (C1-C4)alkyl sulfates, in particular methosulfate, palmitylamidopropyltrimethylammonium (C1-C4)alkyl sulfates, in particular methosulfate; and also mixtures thereof.

Preferentially, the composition according to the invention comprises one or more cationic surfactants chosen from cetyltrimethylammonium halides, in particular chlorides, or behenyltrimethylammonium halides, in particular chlorides, and cetyltrimethylammonium (C1-C4)alkyl sulfates, in particular methosulfates, or behenyltrimethylammonium (C1-C4)alkyl sulfates, in particular methosulfates.

The composition according to the invention preferably comprises the surfactants different from the cationic surfactants of formula (Ia) in a total amount ranging from 0.1% to 10% by weight, better still from 0.2% to 10% by weight, preferentially from 0.5% to 5% by weight, relative to the total weight of the composition. Preferentially, the composition according to the invention preferably comprises the surfactant(s) of formula (Ib), when they are present, in a total amount

ranging from 0.1% to 10% by weight, better still from 0.2% to 10% by weight, preferentially from 0.5% to 5% by weight, relative to the total weight of the composition.

3/ Silicone emulsions

The composition according to the invention also comprises an oil-in-water emulsion having a particle size D50 of less than 350 nm, and comprising:

- a silicone mixture comprising (i) a polydialkylsiloxane comprising trialkylsilyl end groups, having a viscosity at 25°C ranging from 40 000 to 100 000 mPa.s and (ii) an aminosilicone having a viscosity at 25°C ranging from 1000 to 15 000 mPa.s and an amine number ranging from 2 to 10 mg of KOH per gram of aminosilicone;
- a surfactant mixture comprising one or more non-ionic surfactants, said mixture having an HLB ranging from 10 to 16, and
- water.

In the oil-in-water, or silicone-in-water, emulsion, according to the invention, a liquid phase (the dispersed phase) is advantageously dispersed in another liquid phase (the continuous phase); in the present invention, the mixture of silicones, or silicone phase, is dispersed in the aqueous continuous phase.

The mixture of silicones (or silicone mixture) comprises one or more polydialkylsiloxanes comprising trialkylsilyl end groups, preferably of formula (I): R'3SiO(R'2SiO)pSiR'3
in which:

- R', which may be identical or different, is a monovalent hydrocarbon-based radical having from 1 to 18 carbon atoms, preferably from 1 to 6 carbon atoms, better still from 1 to 3 carbon atoms, even better still a methyl radical, and
- p is an integer ranging from 500 to 2000, better still from 1000 to 2000.

The polydialkylsiloxanes comprising trialkylsilyl end groups according to the invention have a viscosity ranging from 40 000 to 100 000 mPa.s (preferably 100 000 excluded) at 25°C, preferably ranging from 40 000 to 70 000 mPa.s at 25°C, better still from 51 000 to 70 000 mPa.s at 25°C.

The polydialkylsiloxanes comprising trialkylsilyl end groups according to the invention are preferably linear, but they may comprise, in addition to the $R'_2SiO_{2/2}$ units (D-units), additional $RSiO_{3/2}$ units (T-units) and/or $SiO_{4/2}$ units (Q-units), in which R', which may be identical or different, is a C1-C18 monovalent hydrocarbon-based radical.

In formula (I), R', which may be identical or different, is preferably:

- an alkyl, preferably C1-C28 alkyl, radical, such as the radicals methyl, ethyl, n-propyl, isopropyl, 1-n-butyl, 2-n-butyl, isobutyl, tert-butyl, n-pentyl, isopentyl, neopentyl, tert-pentyl, hexyl and in particular n-hexyl, heptyl and in particular n-heptyl, octyl and in particular n-octyl, isooctyl, 2,2,4-trimethylpentyl; nonyl and in particular n-nonyl; decyl and in particular n-decyl; dodecyl and in particular n-dodecyl; octadecyl and in particular n-octadecyl;
- an alkenyl radical such as vinyl and allyl;
- a cycloalkyl radical such as cyclopentyl, cyclohexyl, cycloheptyl and methylcyclohexyl;
- an aryl radical such as phenyl, naphthyl, anthryl and phenanthryl;
- an alkaryl radical such as the radicals o-, m- and p-tolyl; xylyl, ethylphenyl;
- an aralkyl radical such as benzyl and phenylethyl.

Preferentially, R' is a methyl radical.

Preferably, the polydialkylsiloxanes comprising trialkylsilyl end groups are polydimethylsiloxanes (PDMSs) comprising trialkylsilyl end groups.

The silicone mixture also comprises one or more aminosilicones, preferably of formula (II): $XR_2Si(OSiAR)_n(OSiR_2)_mOSiR_2X$
in which:

- R, which may be identical or different, is a monovalent hydrocarbon-based radical having from 1 to 18 carbon atoms, preferably from 1 to 6 carbon atoms, better still from 1 to 3 carbon atoms, even better still a methyl radical,
- X, which may be identical or different, represents R or a hydroxyl (OH) or a C1-C6 alkoxy group; preferably X is R, that is to say a monovalent hydrocarbon-based radical having from 1 to 18 carbon atoms, preferably from 1 to 6 carbon atoms, better still from 1 to 3 carbon atoms, even better still a methyl radical,
- A is an amino radical of formula $-R^1-[NR^2-R^3-]xNR^2_2$, or the protonated form of this amino radical, with

- $R^1$ representing a C1-C6 alkylene radical, preferably a $-CH_2CH_2CH_2-$ or $-CH_2CH(CH_3)CH_2-$ radical,
- $R^2$, which may be identical or different, being a hydrogen atom or a C1-C4 alkyl radical, preferably a hydrogen atom,
- $R^3$ being a C1-C6 alkylene radical, preferably a $-CH_2CH_2-$ radical,
- x being 0 or 1;

- m and n are integers such that m+n ranges from 50 to 1000, better still from 50 to 600.

Preferably, A is an amino radical of formula $-R^1-[NR^2-R^3-]xNR^2_2$, or the protonated form of this amino radical, with $R^1$ being $-CH_2CH_2CH_2-$ or $-CH_2CH(CH_3)CH_2-$, $R^2$ being hydrogen atoms, $R^3$ being $-CH_2CH_2-$ and x being equal to 1.
Preferably, R, which may be identical or different, is:

- an alkyl, preferably C1-C28 alkyl, radical, such as the radicals methyl, ethyl, n-propyl, isopropyl, 1-n-butyl, 2-n-butyl, isobutyl, tert-butyl, n-pentyl, isopentyl, neopentyl, tert-pentyl, hexyl and in particular n-hexyl, heptyl and in particular n-heptyl, octyl and in particular n-octyl, isooctyl, 2,2,4-trimethylpentyl; nonyl and in particular n-nonyl; decyl and in particular n-decyl; dodecyl and in particular n-dodecyl; octadecyl and in particular n-octadecyl;
- an alkenyl radical such as vinyl and allyl;
- a cycloalkyl radical such as cyclopentyl, cyclohexyl, cycloheptyl and methylcyclohexyl;
- an aryl radical such as phenyl, naphthyl, anthryl and phenanthryl;
- an alkaryl radical such as the radicals o-, m- and p-tolyl; xylyl, ethylphenyl;
- an aralkyl radical such as benzyl and phenylethyl.

Preferentially, R is a methyl radical.
The aminosilicones according to the invention have a viscosity, measured at 25°C, ranging from 1000 to 15 000 mPa.s, preferably ranging from 1500 to 15 000 mPa.s. The aminosilicones according to the invention have an amine number ranging from 2 to 10 mg of KOH per gram of aminosilicone; preferably from 3.5 to 8 mg.
The molar percentage of amine function is preferably between 0.3 and 8 mol%.
As examples of aminosilicones, mention may be made of aminosilicones comprising trialkylsilyl end groups; preferably aminoethylaminopropylmethylsiloxanes comprising trialkylsilyl end groups, even better still copolymers of aminoethylaminopropylmethylsiloxane comprising trialkylsilyl end groups/dimethylsiloxane.
The amino radical A may be partially or totally protonated, for example by addition of acids to the aminosilicone, so as to obtain the salified form of said amino radical. As acids that may be used, mention may be made of linear or branched carboxylic acids having from 3 to 18 carbon atoms, such as formic acid, acetic acid, propionic acid, butyric acid, pivalic acid, sorbic acid, benzoic acid or salicylic acid. Preferably, the acids may be used in a proportion of from 0.1 to 2.0 mol per mole of amino radical A in the aminosilicone of formula (II).
The silicone mixture preferably comprises (i) one or more polydialkylsiloxanes comprising trialkylsilyl end groups, having a viscosity, at 25°C, ranging from 40 000 to 100 000 mPa.s, in an amount of from 70% to 90% by weight, preferably from 75% to 85% by weight, and (ii) one or more aminosilicones having a viscosity, at 25°C, ranging from 1000 to 15 000 mPa.s and an amine number ranging from 2 to 10 mg of KOH per gram of aminosilicone, in an amount of from 10% to 30% by weight, in particular from 15% to 25% by weight, relative to the total weight of the silicone mixture.
The oil-in-water emulsion also comprises a surfactant mixture which comprises one or more non-ionic surfactants; said surfactant mixture may optionally comprise one or more cationic surfactants.
Said surfactant mixture has an HLB ranging from 10 to 16.
The non-ionic surfactants that may be used may be chosen from alcohols, $\alpha$-diols and (C1-20)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; or else these compounds comprising at least one fatty chain comprising from 8 to 30 carbon atoms and especially from 16 to 30 carbon atoms.
Mention may also be made of condensates of ethylene oxide and of propylene oxide with fatty alcohols; polyethoxylated fatty amides preferably having from 2 to 30 ethylene oxide units, polyglycerolated fatty amides comprising on average from 1 to 5, and in particular from 1.5 to 4, glycerol groups; ethoxylated fatty acid esters of sorbitan preferably containing from 2 to 40 ethylene oxide units, fatty acid esters of sucrose, polyoxyalkylenated and preferably polyoxyethylenated fatty acid esters containing from 2 to 150 mol of ethylene oxide, including oxyethylenated plant oils, N-(C6-C24 alkyl)glucamine derivatives, amine oxides such as (C10-C14 alkyl)amine oxides or N-(C10-C14 acyl)aminopropylmorpholine oxides.
Mention may also be made of non-ionic surfactants of alkyl(poly)glycoside type, represented especially by the following general formula: $R_1O-(R_2O)_t-(G)_v$ in which:

- $R_1$ represents a linear or branched alkyl or alkenyl radical comprising 6 to 24 carbon atoms and especially 8 to 18 carbon atoms, or an alkylphenyl radical of which the linear or branched alkyl radical comprises 6 to 24 carbon atoms and especially 8 to 18 carbon atoms;
- $R^2$ represents an alkylene radical comprising 2 to 4 carbon atoms,
- G represents a sugar unit comprising 5 to 6 carbon atoms,
- t denotes a value ranging from 0 to 10 and preferably 0 to 4,
- v denotes a value ranging from 1 to 15 and preferably 1 to 4.

Preferably, the alkyl(poly)glycoside surfactants are compounds of the formula described above in which:

- $R_1$ denotes a linear or branched, saturated or unsaturated alkyl radical comprising from 8 to 18 carbon atoms,
- $R_2$ represents an alkylene radical comprising 2 to 4 carbon atoms,
- t denotes a value ranging from 0 to 3 and preferably equal to 0,
- G denotes glucose, fructose or galactose, preferably glucose;
- the degree of polymerization, i.e. the value of v, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. C8/C16 alkyl(poly)glucosides 1,4, and in particular decyl glucosides and caprylyl/capryl glucosides, are most particularly preferred.

Among commercial products, mention may be made of the products sold by the company Cognis under the names Plantaren® (600 CS/U, 1200 and 2000) or Plantacare® (818, 1200 and 2000); the products sold by the company SEPPIC under the names Oramix CG 110 and Oramix® NS 10; the products sold by the company BASF under the name Lutensol GD 70, or else the products sold by the company Chem Y under the name AG10 LK.

Preferably, use is made of C8/C16-alkyl (poly)glycosides 1,4, in particular as an aqueous 53% solution, such as those sold by Cognis under the reference Plantacare® 818 UP.

The mono- or polyglycerolated surfactants preferably comprise an average number of glycerol groups ranging from 1 to 30, in particular from 1 to 10, better still from 1.5 to 5. They preferably correspond to one of the following formulae:

$RO[CH2CH(CH2OH)O]_mH$,

$RO[CH2CH(OH)CH2O]_mH$

or

$RO[CH(CH2OH)CH2O]_mH$;

in which:

- R represents a saturated or unsaturated, linear or branched hydrocarbon-based (in particular alkyl or alkenyl) radical comprising 8 to 40 carbon atoms, in particular 10 to 30 carbon atoms, optionally comprising one or more heteroatoms such as O and N; and
- m is an integer ranging from 1 to 30, preferably from 1 to 10, better still from 1.5 to 6.

In particular, R may comprise one or more hydroxyl and/or ether and/or amide groups. Preferably, R is a mono- or polyhydroxylated C10-C20 alkyl or alkenyl radical.

Mention may be made of polyglycerolated (3.5 mol) hydroxylauryl ether, such as the product Chimexane® NF from Chimex.

Mention may also be made of (poly)ethoxylated fatty alcohols preferably comprising one or more saturated or unsaturated, linear or branched hydrocarbon-based chains comprising 8 to 30 carbon atoms, preferably from 12 to 22 carbon atoms, optionally substituted with one or more hydroxyl (OH) groups, in particular 1 to 4 hydroxyl groups.

When the chain is unsaturated, it may comprise one to three conjugated or nonconjugated carbon-carbon double bonds.

The (poly)ethoxylated fatty alcohols preferably correspond to formula (II):

R3-(OCH2CH2)cOH

in which:

- R3 represents a linear or branched alkyl or alkenyl radical comprising 8 to 40 carbon atoms and in particular 8 to 30 carbon atoms, optionally substituted with one or more, in particular 1 to 4, hydroxyl groups; and
- c is an integer ranging from 1 to 200, preferably from 2 to 150, or even from 4 to 50 and even better still from 8 to 30.

The (poly)ethoxylated fatty alcohols are more particularly fatty alcohols comprising 8 to 22 carbon atoms, oxyethylenated with 1 to 30 mol of ethylene oxide (1 to 30 EO); mention may in particular be made of lauryl alcohol 2 EO; lauryl alcohol 3 EO; decyl alcohol 3 EO; decyl alcohol 5 EO and oleyl alcohol 20 EO.

The non-ionic surfactants may advantageously be chosen from:

(i) (poly)oxyalkylenated, in particular (poly)ethoxylated, fatty alcohols, and in particular those of formula: $R_3-(OCH_2CH_2)_cOH$ in which:

- R3 represents a linear or branched alkyl or alkenyl radical comprising 8 to 40 carbon atoms and in particular 8 to 30 carbon atoms, optionally substituted with one or more, in particular 1 to 4, hydroxyl groups; and
- c is an integer ranging from 1 to 200, in particular from 2 to 150, or even from 4 to 50 and even better still from 8 to 20;

(ii) (poly)oxyalkylenated (C8-C32)alkyl phenyl ethers, in particular comprising from 1 to 200, better still from 1 to 30 mol of ethylene oxide;
(iii) polyoxyalkylenated esters of C8-C32 fatty acids and of sorbitan, in particular polyoxyethylenated esters of C8-C32 fatty acids and of sorbitan, preferably having from 2 to 40 ethylene oxide units, better still from 2 to 20 ethylene oxide (EO) units; in particular polyoxyethylenated esters of C10-C24 fatty acids and of sorbitan, preferably having from 2 to 40 ethylene oxide units, better still from 2 to 20 ethylene oxide (EO) units; and
(iv) polyoxyethylenated esters of C8-C32 fatty acids, preferably having from 2 to 150 ethylene oxide units; in particular polyoxyethylenated esters of C10-C24 fatty acids, comprising in particular 2 to 150 ethylene oxide (EO) units.

The non-ionic surfactants may advantageously be chosen from alkyl ethers and alkyl esters of polyalkylene glycol, in particular of polyethylene glycol.

Mention may in particular be made of:

- polyethylene glycol octyl ether; polyethylene glycol lauryl ether; polyethylene glycol tridecyl ether; polyethylene glycol cetyl ether; polyethylene glycol stearyl ether; and most particularly trideceth-3, trideceth-10 and steareth-6;
- polyethylene glycol nonylphenyl ether; polyethylene glycol dodecylphenyl ether; polyethylene glycol cetylphenyl ether; polyethylene glycol stearylphenyl ether;
- polyethylene glycol sorbitan monostearate, polyethylene glycol sorbitan monooleate;
- polyethylene glycol stearate, and in particular PEG100 stearate.

Even better still, the non-ionic surfactants may be chosen from Steareth-6, PEG100 stearate, trideceth-3 and trideceth-10, and mixtures thereof; most particularly, a mixture comprising these four non-ionic surfactants.

The surfactant mixture may optionally comprise one or more cationic surfactants, which may be chosen from tetraalkylammonium, tetraarylammonium and tetraalkylarylammonium salts, in particular halides, and most particularly from cetrimonium or behentrimonium salts, in particular halides, better still chlorides.

The oil-in-water emulsion preferably comprises the surfactant mixture in a total amount ranging from 5% to 15% by weight, in particular from 8% to 15% by weight, even better still from 10% to 12% by weight, relative to the total weight of the emulsion.

The oil-in-water emulsion preferably comprises the non-ionic surfactant(s) in a total amount ranging from 5% to 15% by weight, in particular from 8% to 15% by weight, even better still from 10% to 12% by weight, relative to the total weight of the emulsion.

The oil-in-water emulsion preferably comprises the cationic surfactant(s), when they are present, in a total amount ranging from 0.5% to 1.5% by weight relative to the total weight of the emulsion.

The oil-in-water emulsion preferably comprises the silicone mixture in a total amount ranging from 40% to 60% by weight, in particular from 45% to 55% by weight, relative to the total weight of the emulsion.

The oil-in-water emulsion preferably comprises the polydialkylsiloxane(s) comprising trialkylsilyl end groups in a total amount ranging from 35% to 45% by weight, in particular from 38% to 42% by weight, relative to the total weight of the emulsion. The oil-in-water emulsion preferably comprises the aminosilicone(s) in a total amount ranging from 5% to 15% by weight, in particular from 8% to 12% by weight, relative to the total weight of the emulsion.

The oil-in-water emulsion preferably comprises water in a total amount ranging from 25% to 50% by weight, in particular from 30% to 45% by weight, even better still from 35% to 42% by weight, relative to the total weight of the

emulsion.

The oil-in-water emulsion may also comprise a preservative, such as phenoxyethanol, in an amount ranging from 0.5% to 1% by weight relative to the total weight of the emulsion.

A process for preparing the oil-in-water emulsion preferably comprises:

- a step of mixing one or more polydialkylsiloxanes comprising trialkylsilyl end groups, having a viscosity, at 25°C, ranging from 40 000 to 100 000 mPa.s, and one or more aminosilicones having a viscosity, at 25°C, ranging from 1000 to 15 000 mPa.s and an amine number ranging from 2 to 10 mg of KOH per gram of aminosilicone; at a temperature of from 15°C to 40°C, in particular at 25°C, in order to obtain a fluid mixture of silicones; then
- a step of adding a surfactant mixture comprising one or more non-ionic surfactants, said mixture having an HLB ranging from 10 to 16, to said fluid mixture of silicones, in order to obtain an emulsified silicone mixture; then
- a step of homogenizing said emulsified silicone mixture, followed by
- a step of adding water, in particular demineralized water, preferentially in steps, in order to obtain an oil-in-water emulsion having a particle size D50 of less than 350 nm.

The preparation process may also comprise an additional step of adding one or more preservatives.

The pH of the oil-in-water emulsion is generally between 4 and 6.

The oil-in-water emulsion has a particle size D50 of less than 350 nm, in particular of between 100 and 300 nm, better still between 150 and 250 nm, or even between 160 and 200 nm.

This size corresponds to the average hydrodynamic particle diameter. The particle size D50 is expressed by volume. It can be measured using a ZetaSizer device from Malvern, UK, model Nano-ZS, based on the "Photon Correlation Spectroscopy (PCS)" method.

Method for measuring the particle size

The particle size of the emulsion is measured using a ZetaSizer device from Malvern, UK, model Nano-ZS, based on the "Photon Correlation Spectroscopy (PCS)" method.

The particle size D50 is measured when the evaluation algorithm is "cumulant analysis".

0.5 g of the emulsion is placed in a 250 ml beaker, 100 ml of demineralized water are added and mixing is carried out in order to obtain the solution to be tested. The solution to be tested is placed in the measuring vessel (or cell) and introduced into the measuring device.

The size $D_{50}$ corresponds to the particle diameter value at 50% in cumulative distribution.

For example, if $D_{50}$=170 nm, this means that 50% of the particles have a size of greater than 170 nm, and that 50% of the particles have a size of less than 170 nm. It should be recalled that this distribution is by volume.

Method for measuring the viscosity

The viscosities, in particular of the silicone compounds, are measured at 25°C, 1 atm.

To measure viscosities of between 1000 and 40 000 mPa.s at 25°C, use may be made of an Anton Paar rheometer, model MCR101, cylinder geometry, single gap: CC27 spindle, shear rate1 $S^{-1}$ for 2 minutes, at 25°C.

To measure viscosities of between 40 000 and 100 000 mPa.s at 25°C, use may be made of an Anton Paar rheometer, model MCR101, 25-6 cone (cone-plate geometry, 25 mm in diameter / 6° cone); Zero gap, shear rate 1 $s^{-1}$ for 2 minutes, at 25°C. Three measurements are carried out for each sample, and the viscosity value is taken at 60 seconds. The MCR Rheometer Series products operate according to the USP convention (US Pharmacopeia Convention, 912 - Rotational Rheometer methods).

Method for measuring the amine number

**[0117]** The amine number can be measured by acid-based titration, using a potentiometer [Make: Veego; model VPT-MG].

0.6 g of sample is placed in a 500 ml beaker and a 1:1 toluene-butanol mixture is added, then mixing is carried out.

The solution is titrated with a 0.1 N HCl solution. A determination of the zero value ($V_{blank}$) is also carried out with the 1:1 toluene-butanol mixture alone.

The amine number is calculated by means of the formula:

$$56.11 \times (V - V_{Blank}) \times N / W \text{ mg KOH/ g of sample}$$

With V= volume of HCl required (in ml), $V_{Blank}$= volume of HCl required for the zero value (in ml); N= normality of HCl, i.e. 0.1, and W= weight of the sample (in g).

HLB values

The term HLB relates to the hydrophobicity-lipophilicity balance of a surfactant. It can be measured experimentally or calculated.

In the present application, the HLB values are the values at 25°C.

The HLB values can be calculated by means of the following equation: HLB = (E + P)/5, in which E is the % by weight of oxyethylene and P is the % by weight of polyol, as is described in the publication Griffin, J. Soc. Cosm. Chem. 1954 (vol.5, n°4), pages 249-256.

The HLB values can also be determined experimentally according to the book by Puisieux and Seiller, entitled "Galenica 5: Les systèmes disperses [Galenics 5: Dispersed systems] - Volume I - Agents de surface et emulsions [Surface agents and emulsions] - Chapter IV - Notions de HLB et de HLB critique [Notions of HLB and of critical HLB], pages 153-194 - paragraph 1.1.2. Determination de HLB par voie experimentale [Experimental determination of HLB], pages 164-180".

Preferably, the HLB values that will be taken into account are those obtained by calculation, in particular in the following way: "calculated HLB" = 20 × (molar mass of the hydrophilic part/total molar mass).

Thus, for an oxyethylenated fatty alcohol, the hydrophilic part corresponds to the oxyethylene units fused to the fatty alcohol and the "calculated HLB" then corresponds to the "HLB according to Griffin".

For an ester or an amide, the hydrophilic part is generally defined as being beyond the carbonyl group, starting from the fatty chain(s).

The HLB values of non-ionic surfactants can also be calculated by means of the Davies formula, as described in Davies JT (1957), "A quantitative kinetic theory of emulsion type, I. Physical chemistry of the emulsifying agent", Gas/Liquid and Liquid/Liquid Interface (Proceedings of the International Congress of Surface Activity): 426-438.

According to this formula, the HLB value is obtained by adding the hydrophilic/hydrophobic contribution the to the constituent groups in the surfactant:

$$HLB = (\text{number of hydrophilic groups}) - n(\text{number of groups per } CH_2 \text{ group}) + 7.$$

The HLB values of some cationic surfactants are given in Table IV, in "Cationic emulsifiers in cosmetics", GODFREY, J. Soc. Cosmetic Chemists (1966) 17, pages 17-27.

When two surfactants A and B, of known HLB values, are mixed, the $HLB_{Mix}$ corresponds to the HLB of the mixture and can be expressed by the following equation:

$$HLB_{Mix} = (W_A HLB_A + W_B HLB_B)/ (W_A + W_B)$$

in which $W_A$ is the amount (weight) of the 1st surfactant A and $W_B$ the amount of the 2nd surfactant B, and $HLB_A$ and $HLB_B$ are the HLB values of the surfactant A and of the surfactant B.

Preferably, the composition according to the invention comprises the oil-in-water emulsion in a total amount ranging from 0.1% to 10% by weight, better still from 0.2% to 8% by weight, preferentially from 0.5% to 6% by weight, relative to the total weight of the composition.

Preferably, the composition according to the invention comprises the oil-in-water emulsion in a total amount ranging from 0.1% to 10% by weight, better still from 0.2% to 8% by weight, preferentially from 0.5% to 6% by weight, relative to the total weight of the composition, and the emulsion has a solids (or active material) content of between 40% and 60% by weight, in particular 45% to 55% by weight, relative to the total weight of the emulsion.

4/ Fatty alcohols

The composition according to the invention may also comprise one or more fatty alcohols.

For the purposes of the present invention, the term fatty alcohol is intended to mean an alcohol comprising, in its main chain, at least one saturated or unsaturated, linear or branched hydrocarbon-based chain, such as alkyl or alkenyl, comprising at least 8 carbon atoms, preferably from 8 to 30 carbon atoms, and even better still from 10 to 22 carbon atoms. They are not glycerolated or polyoxyalkylenated.

They preferably have the structure R-OH in which R denotes a linear or branched, saturated or unsaturated C8-C30, preferably C10-C22, better still C12-C24 alkyl or alkenyl group, R possibly being substituted by one or more

hydroxyl groups.

The fatty alcohols may be liquid or solid at ambient temperature (25°C) and at atmospheric pressure (760 mmHg, i.e. $1.013 \times 10^5$ Pa).

The liquid fatty alcohols of the invention may be saturated or unsaturated.

The saturated liquid fatty alcohols are preferably branched. They may optionally comprise in their structure at least one aromatic or non-aromatic ring. Preferably, they are acyclic.

The unsaturated liquid fatty alcohols contain in their structure at least one double or triple bond, and preferably one or more double bonds. When several double bonds are present, there are preferably 2 or 3 of them, and they may be conjugated or unconjugated. These unsaturated liquid fatty alcohols may be linear or branched. They may optionally comprise in their structure at least one aromatic or non-aromatic ring. Preferably, they are acyclic.

The liquid fatty alcohols preferably have the structure R'-OH, in which R' denotes a branched C12-C24 alkyl or linear or branched C12-C24 alkenyl group, R' possibly being substituted by one or more hydroxyl groups. Preferably, R' is a branched C12-C24 alkyl group, optionally substituted with one or more hydroxyl groups; better still, R' does not contain a hydroxyl group.

The solid fatty alcohols are more particularly chosen from linear or branched, saturated or unsaturated alcohols comprising from 8 to 30 carbon atoms and preferably from 8 to 24 carbon atoms.

Preferably, the solid fatty alcohols have the structure R"-OH with R" denoting a linear alkyl group, optionally substituted by one or more hydroxyl groups, comprising from 8 to 30, better still from 10 to 30, or even from 12 to 24 carbon atoms.

They are preferably chosen from saturated or unsaturated, linear or branched, preferably linear and saturated, (mono)alcohols comprising from 8 to 30 carbon atoms, better still from 10 to 30, or even from 12 to 24 carbon atoms.

Preferably, the composition comprises one or more solid fatty alcohols.

As examples of fatty alcohols that may be used in the present invention, mention may be made of oleyl alcohol, linoleyl alcohol, linolenyl alcohol, undecylenyl alcohol, isocetyl alcohol, isostearyl alcohol, 2-octyl-1-dodecanol, 2-butyloctanol, 2-hexyl-1-decanol, 2-decyl-1-tetradecanol, 2-tetradecyl-1-cetanol, cetyl alcohol, stearyl alcohol, cetylstearyl alcohol, and mixtures thereof.

Preferentially, 2-octyl-1-dodecanol, oleyl alcohol, cetyl alcohol, stearyl alcohol, cetylstearyl alcohol, and mixtures thereof, may be used.

The composition according to the invention can comprise the fatty alcohol(s) in a total amount ranging from 0.1% to 20% by weight, better still from 0.5% to 15% by weight and preferentially from 1% to 10% by weight, relative to the total weight of the composition.

5/ Other ingredients

The composition according to the invention can also comprise one or more additional fatty substances, different from the silicones present in the silicone emulsion above, and from the fatty alcohols above, preferably one or more non-silicone fatty substances which are liquid or solid at 25°C, 1 atm., in particular chosen from liquid or solid fatty esters.

The term "fatty ester" is intended to mean an ester comprising one or more linear or branched, saturated or unsaturated hydrocarbon-based chains comprising from 6 to 30 carbon atoms, optionally substituted in particular with one or more hydroxyl groups (in particular 1 to 4). If they are unsaturated, these compounds may comprise one to three conjugated or unconjugated carbon-carbon double bonds.

Mention may in particular be made of the esters of saturated or unsaturated, linear or branched C1-C26 aliphatic mono- or polyacids and of saturated or unsaturated, linear or branched C1-C26 aliphatic mono- or polyalcohols, the total carbon number of the esters being more particularly greater than or equal to 10.

Preferably, for the esters of monoalcohols, at least one from among the alcohol and the acid from which the esters of the invention are derived is branched.

Among the monoesters of monoacids and of monoalcohols, mention may be made of alkyl, in particular C1-C28 alkyl, palmitates, such as ethyl palmitate or isopropyl palmitate, alkyl, in particular C1-C28 alkyl, myristates such as isopropyl myristate or ethyl myristate, isocetyl stearate, 2-ethylhexyl isononanoate, isodecyl neopentanoate and isostearyl neopentanoate.

Esters of C4-C22 dicarboxylic or tricarboxylic acids and of C1-C22 alcohols and esters of mono-, di- or tricarboxylic acids and of di-, tri-, tetra- or pentahydroxy alcohols which are C2-C26 may also be used.

Mention may be made especially of: diethyl sebacate; diisopropyl sebacate; diisopropyl adipate; di-n-propyl adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol

distearates.

Mention may also be made of esters of sugars and of linear or branched, saturated or unsaturated $C_6$-$C_{30}$, preferably C12-C22, fatty acids

If they are unsaturated, these compounds may comprise one to three conjugated or unconjugated carbon-carbon double bonds.

The term "sugar" is intended to mean oxygen-bearing hydrocarbon-based compounds containing several alcohol functions, with or without aldehyde or ketone functions, and which comprise at least 4 carbon atoms. These sugars may be monosaccharides, oligosaccharides or polysaccharides. Preferably, these sugars are chosen from sucrose, glucose, galactose, ribose, fucose, maltose, fructose, mannose, arabinose, xylose and lactose, and derivatives thereof, especially alkyl derivatives, such as methyl derivatives, for instance methylglucose.

The esters according to this variant may be mono-, di-, tri- and tetraesters, or polyesters.

These esters may be, for example, oleates, laurates, palmitates, myristates, behenates, cocoates, stearates, linoleates, linolenates, caprates and arachidonates, and mixtures thereof, such as, especially, oleopalmitate, oleostearate or palmitostearate mixed esters.

More particularly, use is made of monoesters and diesters and especially of sucrose, glucose or methylglucose mono- or dioleates, stearates, behenates, oleopalmitates, linoleates, linolenates or oleostearates, or alternatively of methylglucose dioleate (Glucate® DO).

It is also possible to use pentaerythrityl esters, preferably pentaerythrityl tetraisostearate, pentaerythrityl tetraoctanoate, and caprylic and capric acid hexaesters as a mixture with dipentaerythritol.

Among the natural or synthetic monoacid, diacid or triacid esters of glycerol, mention may be made of plant oils or synthetic oils.

More particularly, said plant oils or synthetic oils are chosen from triglyceride oils of plant or synthetic origin, such as liquid fatty acid triglycerides containing from 6 to 30 carbon atoms, for instance heptanoic or octanoic acid triglycerides, or alternatively, for example, sesame oil, soybean oil, coffee oil, safflower oil, borage oil, sunflower oil, olive oil, apricot kernel oil, camellia oil, bambara pea oil, avocado oil, mango oil, rice bran oil, cottonseed oil, rose oil, kiwi seed oil, sea buckthorn pulp oil, blueberry seed oil, poppy seed oil, orange pip oil, sweet almond oil, palm oil, coconut oil, vernonia oil, marjoram oil, baobab oil, rapeseed oil, ximenia oil, pracaxi oil, caprylic/capric acid triglycerides such as those sold by the company Stearineries Dubois or those sold under the names Miglyol® 810, 812 and 818 by the company Dynamit Nobel, jojoba oil and shea butter oil.

Mention may preferably be made of triglycerides of plant origin, in particular oils chosen from avocado oil, olive oil, camellia oil and apricot kernel oil, and mixtures thereof, and $C_4$-$C_{22}$ dicarboxylic or tricarboxylic acid esters of $C_1$-$C_{22}$ alcohols, in particular 1,3-propanediol dicaprylate.

Preferentially, the esters may be chosen from ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, butyl stearate, isobutyl stearate; dioctyl malate, hexyl laurate, 2-hexyldecyl laurate, isononyl isononanoate or cetyl octanoate.

The care composition may comprise the fatty substance(s), when they are present, in a content ranging from 0.01% to 20% by weight, preferably from 0.05% to 15% by weight and preferentially from 0.1% to 10% by weight, or even from 0.2% to 5% by weight, relative to the total weight of the composition.

The composition according to the invention is advantageously aqueous with a water content of preferably between 40% and 98% by weight, in particular between 50% and 98% by weight, or even between 60% and 95% by weight, preferentially between 70% and 95% by weight, relative to the total weight of the composition.

The pH of composition is preferably between 2.5 and 6, in particular between 2.5 and 5, or even between 2.8 and 4.

The composition according to the invention may also comprise one or more additives chosen from anionic, non-ionic, cationic or amphoteric polymers; ceramides, pseudo-ceramides, vitamins and provitamins including panthenol, water-soluble and liposoluble sunscreens, nacreous agents and opacifiers, sequestrants, solubilizers, antioxidants, antidandruff agents, anti-seborrhoeic agents, agents for preventing hair loss and/or for promoting hair regrowth, penetrants, fragrances, peptizers and preservatives. These additives may be present in the composition according to the invention in an amount ranging from 0 to 20% by weight, relative to the total weight of the composition. Those skilled in the art will take care to select these optional additives and the amounts thereof so that they do not harm the properties of the compositions of the present invention.

The compositions according to the invention may advantageously be in the form of a hair composition, in particular in the form of a rinse-off conditioner, of a mask or of a leave-on care product.

The present invention also relates to a cosmetic process for treating, more particularly for conditioning, keratin materials, and in particular keratin fibres, and in particular the hair, which comprises the application to said keratin materials of a composition as described above, optionally followed by a leave-on time and/or by a rinsing step and/or by a drying step.

The application may be performed on dry or wet hair, preferably wet hair.

The leave-on time of the composition on the keratin fibres may range from 5 seconds to 10 minutes, better still from 10 seconds to 5 minutes and even better still from 20 seconds to 2 minutes.

This application may or may not be followed by rinsing, and is preferably followed by rinsing.

The invention is illustrated in greater detail by the following examples. Unless otherwise indicated, the amounts indicated are expressed in % by weight of active material (AM) relative to the total weight of the composition.

**Example** 1: **Preparation of the silicone emulsion**

450 g of fluid aminosilicone (copolymer of dimethylsiloxane - aminoethylaminopropylmethylsiloxane comprising trimethylsilyl end groups, having an amine number of 7.2 mg of KOH/g and a viscosity of 5600 mPa.s at 25°C) are transferred into a 1st vessel; 1800 g of dimethylsiloxane comprising trimethylsilyl end groups, having a viscosity of 61 500 mPa.s at 25°C, are added, with stirring, and the stirring is maintained for 2 hours at ambient temperature.

In a separate vessel, 49 g of steareth-6 and 62 g of PEG100 stearate are mixed, and the mixture is heated to 60°C. The mixture is maintained at this temperature until a liquid mixture is obtained, then 31 g of trideceth-3 and 350 g of trideceth-10 (80% of active material) are added. The surfactant mixture has an HLB = 11.25. 80 g of water and 6.2 g of glacial acetic acid are added and the stirring is continued until a creamy paste is obtained.

The content of this 2nd vessel (creamy paste) is then transferred into the 1st vessel (containing the silicones), then the mixture obtained is mixed for 30 minutes at ambient temperature (20-25°C). The mixing steps are carried out in order to obtain a homogeneous mixture; they are carried out at ambient temperature.

79.6 g of demineralized water are added and mixing is carried out for 60 minutes. 72.7 g of demineralized water are added and mixing is carried out for 50 minutes. 197.4 g of demineralized water are added and mixing is carried out for 5 minutes. 294.3 g of demineralized water are added and mixing is carried out for 5 minutes. 180 g of demineralized water are added and mixing is carried out for 5 minutes. 180 g of demineralized water are added and mixing is carried out for 5 minutes. 197.4 g of demineralized water are added and mixing is carried out for 5 minutes. 197.4 g of demineralized water are added and mixing is carried out for 3 minutes. 228.5 g of demineralized water are added and mixing is carried out for 3 minutes. Finally, 40.5 g of 2-phenoxyethanol (preservative) are added and mixing is carried out for 3 minutes.

An oil-in-water emulsion having a particle size $D_{50}$ of 170 nm is obtained.

**Example 2**

The cosmetic compositions below comprising the following ingredients (amounts expressed as % by weight of active material AM, unless otherwise indicated) are prepared:

| Ingredients | Invention A | Comparative B | Comparative C |
|---|---|---|---|
| Cetearyl alcohol | 6% | 6% | 6% |
| Mixture of myristyl stearate and myristyl palmitate (INCI: CETYL ESTERS) | 0.5% | 0.5% | 0.5% |
| Silicone emulsion as prepared in Example 1 | 2% (0.8% AM of silicone + 0.2% AM of aminosilicone) | 2% (0.8% AM of silicone + 0.2% AM of aminosilicone) | 2% (0.8% AM of silicone + 0.2% AM of aminosilicone) |
| Dicetyldimethylammonium chloride (VARISOFT 432 CG from Evonik) | 1%AM | 2% AM | - |
| Cetyltrimethylammonium chloride (DEHYQUART A OR from BASF) | 1%AM | - | 2% AM |
| Preservative, fragrance | qs | qs | qs |
| Water | qs 100% | qs 100% | qs 100% |

Compositions are obtained which can be used as rinse-off conditioner.

a) Viscosity

The viscosity of the 3 compositions was measured 24 h after production, then after two months of storage at ambient temperature (25°C) (Rheomat RM 200, 25°C, spindle 3, 200 rpm).
The results obtained are given below:

| | Viscosity 24 h after production | Viscosity after 2 months at 25°C | % variation |
|---|---|---|---|
| A (invention) | 1145 cps | 1270 cps | +10.9% |
| B (comparative) | 815 cps | 650 cps | -20.2% |
| C (comparative) | 550 cps | 690 cps | +25.5% |

The composition A according to the invention has a higher viscosity than the comparative compositions B and C, which allows easy application without the risk of running.
After a storage period of 2 months at 25°C, the change in the viscosity, compared with the initial viscosity, is smaller for the composition A according to the invention compared with the compositions B and C.

b) Deposit of fatty substances and of cations

The compositions A, B and C were applied to pads of hair trimmings weighing 200 mg, previously washed with a standard shampoo and rinsed, in a proportion of 1 ml of composition per pad of hair.
After rinsing and drying, the deposit of, on the one hand, fatty substances and of, on the other hand, cations was measured by gas chromatography after desorption in dichloromethane.
The following results are obtained (in micrograms/gram of hair)

| | Total fatty substances (μg/g hair) | Total cations (μg/g hair) |
|---|---|---|
| A (invention) | 2925 | 755 |
| B (comparative) | 7143 | 713 |
| C (comparative) | 2328 | 1197 |

It is noted that the composition A makes it possible to deposit amounts of fatty substances and of cations that are intermediate between the composition B and the composition C.
Excessively large deposits of fatty substances result in an unclean greasy feel, contrary to the desired natural and clean feel.
Excessively large deposits of cations result in a softening of the fibres, contrary to the desired tonicity effect.
These results are confirmed by the rubbing and bending force measurements, carried out on locks, below.

c) Rubbing

Locks of hair, washed beforehand with a standard shampoo and rinsed, are treated with each of the compositions A, B and C, in a proportion of 0.4 g of composition per g of hair.
After rinsing and drying, 4 lock portions, each weighing 150 milligrams, are sampled from each lock, and are each mounted on a support.
Each lock portion is then inserted between 2 jaws: the maximum force required to move the support along the 2 jaws is then recorded.
The mean force corresponding to the mean of the 4 maximum forces measured is calculated.
The lower the rubbing force, the more representative is the surface of the lock of an unclean greasy feel.
The following results are obtained:

| | Mean rubbing force (in N) |
|---|---|
| A (invention) | 0.334 |
| B (comparative) | 0.143 |
| C (comparative) | 0.147 |

The rubbing forces of the compositions B and C are lower than that of the composition A; this reflects a surface that is much more representative of an unclean greasy feel for the compositions B and C.

d) Bending

Locks of hair, washed beforehand with a standard shampoo and rinsed, are treated with each of the compositions A, B and C, in a proportion of 0.4 g of composition per g of hair.
After rinsing and drying, 4 lock portions, weighing 150 milligrams, are sampled from each lock, and are each mounted on a support.
Each support is then moved perpendicularly towards 2 stops: the maximum force required to continue the movement is recorded.
The mean force corresponding to the mean of the 4 maximum forces measured is calculated.
The greater the bending, the more resistant/tonic is the hair.
The following results are obtained:

| | Mean bending force (in N) |
|---|---|
| A (invention) | 0.0329 |
| B (comparative) | 0.0258 |
| C (comparative) | 0.0257 |

The composition A results in a bending force greater than that of the compositions B and C, which reflects a better bending resistance of the hair, and therefore a better tonicity.
In conclusion, only the composition A according to the invention makes it possible to produce a feel that is soft while at the same time remaining clean, and also allows the hair to retain tonicity.

**Example 3**

The cosmetic compositions below comprising the following ingredients (amounts expressed as % by weight of active material AM, unless otherwise indicated) are prepared:

| Ingredients | Invention A | Comparative A' |
|---|---|---|
| Cetearyl alcohol | 6% | 6% |
| Mixture of myristyl stearate and myristyl palmitate (INCI: CETYL ESTERS) | 0.5% | 0.5% |
| Silicone emulsion as prepared in Example 1 (silicone + aminosilicone + nonionic surfactants) | 2% (0.8% AM of silicone + 0.2% AM of aminosilicone) | - |
| Dimethicone ((Xiameter PMX-200 Silicone Fluid) | - | 0,8% am |
| Amodimethicone (DC2-8566 Amino Fluid) | - | 0,2% am |
| Non-ionic surfactants (laureth-11 + PEG-100 stearate + steareth-10 + laureth-3) | - | 0,176% am |
| Dicetyldimethylammonium chloride (VARISOFT 432 CG from Evonik) | 1%AM | 1%AM |
| Cetyltrimethylammonium chloride (DEHYQUART A OR from BASF) | 1%AM | 1%AM |
| Preservative, fragrance | qs | qs |
| Water | qs 100% | qs 100% |

The compositions A and A' were applied to pads of natural hair trimmings weighing 100 mg, previously washed with a standard shampoo and rinsed, in a proportion of 1 ml of composition per pad of hair.

After rinsing and drying, the silicon (Si) deposition has been measured, after the hair has been ground and homogenized, via the measurement of elemental Silicon by X-ray fluorescence spectrometry using a spectrometer S8 TIGER WDXRF from BRUKER (Wavelength Dispersion) XRF system.

The following results are obtained (in micrograms/gram of hair)

| | Total Si (μg/g hair) |
|---|---|
| A (invention) | 166 |
| A' (comparative) | 554 |

It is noted that the invention composition A makes it possible to deposit amounts of silicone moderate in comparison with comparative composition A'.

As excessively large deposits of silicone result in an unclean greasy feel (loaded or charged touch), this is unappreciated by consumers.

With the invention composition, the deposit of silicone is thinner, and the feel (touch) is more natural and more clean (clean feel).

## Claims

**1.** Cosmetic composition, in particular hair composition, comprising:

(i) one or more cationic surfactants of formula (Ia):

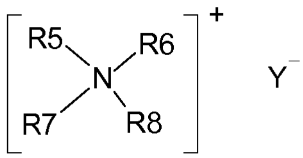

(Ia)

in which:

- R5 and R6, which may be identical or different, represent a linear or branched, saturated or unsaturated aliphatic group comprising from 8 to 30 carbon atoms, preferably from 12 to 24 carbon atoms, said aliphatic group possibly comprising one or more heteroatoms such as oxygen, nitrogen, sulfur and/or halogens; or an aromatic group such as an aryl or alkaryl group, in particular a benzyl group;
- R7 and R8, which may be identical or different, represent a linear or branched aliphatic group comprising from 1 to 6 carbon atoms;
- $Y^-$ is an anion;

(ii) one or more cationic surfactants other than the cationic surfactants of formula (Ia), and
(iii) an oil-in-water emulsion having a particle size D50 of less than 350 nm, and comprising:

- a silicone mixture comprising (i) a polydialkylsiloxane comprising trialkylsilyl end groups, having a viscosity at 25°C ranging from 40 000 to 100 000 mPa.s and (ii) an aminosilicone having a viscosity at 25°C ranging from 1000 to 15 000 mPa.s and an amine number ranging from 2 to 10 mg of KOH per gram of aminosilicone;
- a surfactant mixture comprising one or more non-ionic surfactants, said mixture having an HLB ranging from 10 to 16, and
- water,

wherein the particle size, the viscosity , the amine number and the HLB values are determined according to the methods specified in the description.

**2.** Composition according to Claim 1, comprising one or more cationic surfactants of formula (Ia) in which:

- R5 represents a saturated linear C8-C30, in particular C12-C24, alkyl group, or a (C12-C22)alkylamido(C2-C6)alkyl group; preferentially, a behenyl, cetyl, stearyl or stearamidopropyl radical; and/or

- R6 represents a saturated linear C8-C30, in particular C12-C24, alkyl group, or a (C12-C22)alkyl acetate group; preferentially, a benzyl, behenyl, cetyl, stearyl or myristyl acetate radical; and/or
- R7 and R8, which may be identical or different, represent a saturated linear alkyl group comprising from 1 to 6 carbon atoms, in particular from 1 to 3 carbon atoms; preferentially a methyl group.

**3.** Composition according to either of the preceding claims, comprising one or more cationic surfactants of formula (Ia) chosen from di(C8-C30)alkyldimethylammonium salts, and in particular distearyldimethylammonium salts, dicetyldimethylammonium salts, benzyldimethylstearylammonium salts, stearamidopropyldimethyl(myristyl acetate)ammonium salts, and mixtures thereof; more particularly from di(C8-C30)alkyldimethylammonium halides, in particular chlorides, and in particular distearyldimethylammonium halides, in particular chlorides, dicetyldimethylammonium halides, in particular chlorides, benzyldimethylstearylammonium halides, in particular chlorides, stearamidopropyldimethyl(myristyl acetate)ammonium halides, in particular chlorides; or else from di(C8-C30)alkyldimethylammonium (C1-C4)alkyl sulfates, in particular methosulfates, and in particular distearyldimethylammonium (C1-C4)alkyl sulfates, in particular methosulfates, dicetyldimethylammonium(C1-C4)alkyl sulfates, in particular methosulfates, benzyldimethylstearylammonium (C1-C4)alkyl sulfates, in particular methosulfates, stearamidopropyldimethyl(myristyl acetate)ammonium (C1-C4)alkyl sulfates, in particular methosulfates; and also mixtures thereof; and preferentially from dicetyldimethylammonium halides, in particular chlorides, or dicetyldimethylammonium (C1-C4)alkyl sulfates, in particular methosulfates.

**4.** Composition according to one of the preceding claims, comprises the surfactant(s) of formula (Ia) in a total amount ranging from 0.1% to 10% by weight, better still from 0.2% to 10% by weight, preferentially from 0.5% to 5% by weight, relative to the total weight of the composition.

**5.** Composition according to one of the preceding claims, in which the cationic surfactant(s) different from the cationic surfactants of formula (Ia) are chosen from the cationic surfactants of formula (Ib):

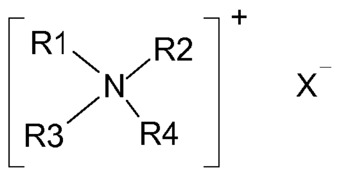

(Ib)

in which:

- R1 represents a linear or branched, saturated or unsaturated aliphatic group comprising from 8 to 30 carbon atoms, preferably from 12 to 24 carbon atoms, said aliphatic group possibly comprising one or more heteroatoms such as oxygen, nitrogen, sulfur and/or halogens; preferably a linear or branched, saturated aliphatic group comprising from 8 to 30 carbon atoms, preferably from 12 to 24 carbon atoms, said aliphatic group possibly comprising one or more heteroatoms such as oxygen and/or nitrogen; even better still a saturated linear C8-C30, in particular C12-C24, alkyl group or a (C12-C22)alkylamido(C2-C6)alkyl group; preferentially a behenyl, cetyl or palmitylamidopropyl radical,
- R2 to R4, which may be identical or different, represent a linear or branched, saturated or unsaturated aliphatic group comprising from 1 to 6 carbon atoms; in particular, a saturated linear alkyl group comprising 1 to 6 carbon atoms, better still 1 to 3 carbon atoms; preferentially, a methyl group,
- $X^-$ is an anion, in particular chosen from halides such as chloride, bromide and iodide; phosphates, acetates, lactates, (C1-C4)alkyl sulfates, (C1-C4)alkyl- or (C1-C4)alkylarylsulfonates.

**6.** Composition according to the preceding claim, in which the cationic surfactant(s) of formula (Ib) are chosen from (C8-C30)alkyltrimethylammonium salts, and in particular cetyltrimethylammonium salts, behenyltrimethylammonium salts, palmitylamidopropyltrimethylammonium salts and mixtures thereof; more particularly from (C8-C30)alkyltrimethylammonium halides, in particular chlorides, and in particular cetyltrimethylammonium halides, in particular chloride, behenyltrimethylammonium halides, in particular chloride, palmitylamidopropyltrimethylammonium halides, in particular chloride; or (C8-C30)alkyltrimethylammonium (C1-C4)alkyl sulfates, in particular methosulfates, and in particular cetyltrimethylammonium (C1-C4)alkyl sulfates, in particular methosulfate, behenyltrimethylammonium (C1-C4)alkyl sulfates, in particular methosulfate, palmitylamidopropyltrimethylammonium (C1-C4)alkyl sulfates, in particular methosulfate; and also mixtures thereof; and preferentially from cetyltrimethylammonium halides, in particular chlorides, or behenyltrimethylammonium halides, in particular chlorides, and cetyltrimethylammonium (C1-C4)alkyl sulfates, in particular methosulfates, or behenyltrimethylammonium (C1-C4)alkyl sulfates, in particular methosulfates.

**7.** Composition according to one of the preceding claims, comprising the surfactant(s) different from the cationic surfactants of formula (Ia) in a total amount ranging from 0.1% to 10% by weight, better still from 0.2% to 10% by weight, preferentially from 0.5% to 5% by weight, relative to the total weight of the composition.

**8.** Composition according to either of Claims 5 and 6, comprising the surfactant(s) of formula (Ib) in a total amount ranging from 0.1% to 10% by weight, better still from 0.2% to 10% by weight, preferentially from 0.5% to 5% by weight, relative to the total weight of the composition.

**9.** Composition according to one of the preceding claims, in which the silicone mixture comprises one or more polydialkylsiloxanes comprising trialkylsilyl end groups of formula (I): R'3SiO(R'2SiO)pSiR'3
in which:

- R', which may be identical or different, is a monovalent hydrocarbon-based radical having from 1 to 18 carbon atoms, preferably from 1 to 6 carbon atoms, better still from 1 to 3 carbon atoms, even better still a methyl radical, and
- p is an integer ranging from 500 to 2000, better still from 1000 to 2000;

preferably having a viscosity ranging from 40 000 to 100 000 mPa.s at 25°C, preferably ranging from 40 000 to 70 000 mPa.s at 25°C, better still from 51 000 to 70 000 mPa.s at 25°C.

**10.** Composition according to one of the preceding claims, in which the silicone mixture comprises one or more aminosilicones of formula (II):

$$XR_2Si(OSiAR)_n(OSiR_2)_mOSiR_2X$$

in which:

- R, which may be identical or different, is a monovalent hydrocarbon-based radical having from 1 to 18 carbon atoms, preferably from 1 to 6 carbon atoms, better still from 1 to 3 carbon atoms, even better still a methyl radical,
- X, which may be identical or different, represents R or a hydroxyl (OH) or a C1-C6 alkoxy group; preferably X is R, that is to say a monovalent hydrocarbon-based radical having from 1 to 18 carbon atoms, preferably from 1 to 6 carbon atoms, better still from 1 to 3 carbon atoms, even better still a methyl radical,
- A is an amino radical of formula $-R^1-[NR^2-R^3-]xNR^2_2$, or the protonated form of this amino radical, with

  - $R^1$ representing a C1-C6 alkylene radical, preferably a $-CH_2CH_2CH_2-$ or $-CH_2CH(CH_3)CH_2-$ radical,
  - $R^2$, which may be identical or different, being a hydrogen atom or a C1-C4 alkyl radical, preferably a hydrogen atom,
  - $R^3$ being a C1-C6 alkylene radical, preferably a $-CH_2CH_2-$ radical,
  - x being 0 or 1;

- m and n are integers such that m+n ranges from 50 to 1000, better still from 50 to 600;

preferably having a viscosity at 25°C ranging from 1000 to 15 000 mPa.s, preferably from 1500 to 15 000 mPa.s, and/or an amine number ranging from 2 to 10 mg of KOH per gram of aminosilicone, preferably from 3.5 to 8 mg.

**11.** Composition according to one of the preceding claims, comprising (i) one or more polydialkylsiloxanes comprising trialkylsilyl end groups, having a viscosity, at 25°C, ranging from 40 000 to 100 000 mPa.s, in an amount of from 70% to 90% by weight, preferably from 75% to 85% by weight, and (ii) one or more aminosilicones having a viscosity, at 25°C, ranging from 1000 to 15 000 mPa.s and an amine number ranging from 2 to 10 mg of KOH per gram of aminosilicone, in an amount of from 10% to 30% by weight, in particular from 15% to 25% by weight, relative to the total weight of the silicone mixture.

**12.** Composition according to one of the preceding claims, in which the surfactant mixture comprises one or more nonionic surfactants chosen from:

(i) (poly)oxyalkylenated, in particular (poly)ethoxylated, fatty alcohols, and in particular those of formula: $R_3$-$(OCH_2CH_2)_cOH$ in which:

- R3 represents a linear or branched alkyl or alkenyl radical comprising 8 to 40 carbon atoms and in particular 8 to 30 carbon atoms, optionally substituted with one or more, in particular 1 to 4, hydroxyl groups; and
- c is an integer ranging from 1 to 200, in particular from 2 to 150, or even from 4 to 50 and even better still from 8 to 20;

(ii) (poly)oxyalkylenated (C8-C32)alkyl phenyl ethers, in particular comprising from 1 to 200, better still from 1 to 30 mol of ethylene oxide;
(iii) polyoxyalkylenated esters of C8-C32 fatty acids and of sorbitan, in particular polyoxyethylenated esters of C8-C32 fatty acids and of sorbitan, preferably having from 2 to 40 ethylene oxide units, better still from 2 to 20 ethylene oxide (EO) units; in particular polyoxyethylenated esters of C10-C24 fatty acids and of sorbitan, preferably having from 2 to 40 ethylene oxide units, better still from 2 to 20 ethylene oxide (EO) units; and
(iv) polyoxyethylenated esters of C8-C32 fatty acids, preferably having from 2 to 150 ethylene oxide units; in particular polyoxyethylenated esters of C10-C24 fatty acids, comprising in particular 2 to 150 ethylene oxide (EO) units.

**13.** Composition according to one of the preceding claims, in which the oil-in-water emulsion comprises:

- the surfactant mixture in a total amount ranging from 5% to 15% by weight, in particular from 8% to 15% by weight, even better still from 10% to 12% by weight, relative to the total weight of the emulsion; and/or
- the non-ionic surfactant(s) in a total amount ranging from 5% to 15% by weight, in particular from 8% to 15% by weight, even better still from 10% to 12% by weight, relative to the total weight of the emulsion;
- the silicone mixture in a total amount ranging from 40% to 60% by weight, in particular from 45% to 55% by weight, relative to the total weight of the emulsion; and/or
- the polydialkylsiloxane(s) comprising trialkylsilyl end groups, in a total amount ranging from 35% to 45% by weight, in particular from 38% to 42% by weight, relative to the total weight of the emulsion; and/or
- the aminosilicone(s) in a total amount ranging from 5% to 15% by weight, in particular from 8% to 12% by weight, relative to the total weight of the emulsion; and/or
- water in a total amount ranging from 25% to 50% by weight, in particular from 30% to 45% by weight, even better still from 35% to 42% by weight, relative to the total weight of the emulsion.

**14.** Composition according to one of the preceding claims, in which the oil-in-water emulsion has a particle size D50 of between 100 and 300 nm, better still between 150 and 250 nm, or even between 160 and 200 nm.

**15.** Composition according to one of the preceding claims, comprising the oil-in-water emulsion in a total amount ranging from 0.1% to 10% by weight, better still from 0.2% to 8% by weight, preferentially from 0.5% to 6% by weight, relative to the total weight of the composition.

**16.** Composition according to one of the preceding claims, also comprising one or more fatty alcohols, in particular of structure R-OH in which R denotes a linear or branched, saturated or unsaturated C8-C30, preferably C10-C22, better still C12-C24, alkyl or alkenyl group, R possibly being substituted with one or more hydroxyl groups; preferably in a total amount ranging from 0.1 % to 20% by weight, better still from 0.5% to 15% by weight, preferentially from 1% to 10% by weight, relative to the total weight of the composition.

**17.** Cosmetic process for treating, more particularly for conditioning, keratin materials, and in particular keratin fibres, and in particular the hair, which comprises the application to said keratin materials of a composition according to one of the preceding claims, optionally followed by a leave-on time and/or by a rinsing step and/or by a drying step.

**Patentansprüche**

**1.** Kosmetische Zusammensetzung, insbesondere Haarzusammensetzung, umfassend:

(i) ein oder mehrere kationische Tenside mit der Formel (Ia):

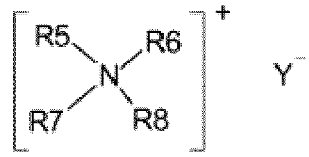

(Ia)

worin:

- R5 und R6, die identisch oder unterschiedlich sein können, für eine lineare oder verzweigte, gesättigte oder ungesättigte aliphatische Gruppe, die 8 bis 30 Kohlenstoffatome, vorzugsweise 12 bis 24 Kohlenstoffatome umfasst, wobei die aliphatische Gruppe möglicherweise ein oder mehrere Heteroatome umfasst, wie Sauerstoff, Stickstoff, Schwefel und/oder Halogene; oder eine Aryl- oder Aralkylgruppe stehen, insbesondere eine Benzylgruppe;
- R7 und R8, die identisch oder unterschiedlich sein können, für eine lineare oder verzweigte aliphatische Gruppe stehen, die 1 bis 6 Kohlenstoffatome umfasst;
- $Y^-$ ein Anion ist;

(ii) ein oder mehrere kationische Tenside, die von den kationischen Tensiden der Formel (Ia) verschieden sind, und
(iii) eine Öl-in-Wasser-Emulsion mit einer Partikelgröße D50 von weniger als 350 nm, und umfassend:

- eine Silikonmischung, umfassend (i) ein Polydialkylsiloxan, das Trialkylsilylendgruppen umfasst, mit einer Viskosität bei 25 °C im Bereich von 40 000 bis 100 000 mPa·s, und (ii) ein Aminosilikon mit einer Viskosität bei 25 °C im Bereich von 1000 bis 15 000 mPa·s und einer Aminzahl im Bereich von 2 bis 10 mg KOH pro Gramm Aminosilikon;
- eine Tensidmischung, umfassend ein oder mehrere nicht-ionische Tenside, wobei die Mischung einen HLB-Wert im Bereich von 10 bis 16 hat, und
- Wasser,

wobei die Partikelgröße, die Viskosität, die Aminzahl und die HLB-Werte gemäß den in den technischen Angaben angegebenen Verfahren bestimmt werden.

**2.** Zusammensetzung nach Anspruch 1, umfassend ein oder mehrere kationische Tenside der Formel (Ia), worin:

- R5 für eine gesättigte lineare $C_8$-$C_{30}$-, insbesondere $C_{12}$-$C_{24}$-Alkylgruppe oder eine ($C_{12}$-$C_{22}$) -Alkylamido ($C_2$-$C_6$) alkylgruppe steht, vorzugsweise einen Behenyl-, Cetyl-, Stearyl- oder Stearamidopropylrest; und/oder
- R6 für eine gesättigte lineare $C_8$-$C_{30}$-, insbesondere $C_{12}$-$C_{24}$-Alkylgruppe oder eine ($C_{12}$-$C_{22}$) -Alkylacetatgruppe steht, vorzugsweise einen Benzyl-, Behenyl-, Cetyl-, Stearyl- oder Myristylacetatrest; und/oder
- R7 und R8, die identisch oder unterschiedlich sein können, für eine gesättigte lineare Alkylgruppe, die 1 bis 6 Kohlenstoffatome, insbesondere 1 bis 3 Kohlenstoffatome umfasst; vorzugsweise eine Methylgruppe stehen.

**3.** Zusammensetzung nach einem der vorhergehenden Ansprüche, umfassend ein oder mehrere kationische Tenside der Formel (Ia) ausgewählt aus Di ($C_8$-$C_{30}$) alkyldimethylammoniumsalzen und insbesondere Distearyldimethylammoniumsalzen, Dicetyldimethylammoniumsalzen, Benzyldimethylstearylammoniumsalzen, Stearamidopropyldimethyl(myristylacetat)ammoniumsalzen und Mischungen davon; insbesondere aus Di($C_8$-$C_{30}$)alkyldimethylammoniumhalogeniden, insbesondere - chloriden, und insbesondere Distearyldimethylammoniumhalogeniden, insbesondere -chloriden, Dicetyldimethylammoniumhalogeniden, insbesondere -chloriden, Benzyldimethylstearylammoniumhalogeniden, insbesondere -chloriden, Stearamidopropyl-dimethyl(myristylacetat)ammoniumhalogeniden, insbesondere -chloriden; oder ansonsten aus Di($C_8$-$C_{30}$) alkyldimethylammonium($C_1$-$C_4$) alkylsulfaten, insbesondere -methosulfaten, und insbesondere Distearyldimethylammonium($C_1$-$C_4$)alkylsulfaten, insbesondere - methosulfaten, Dicetyldimethylammonium($C_1$-$C_4$)-alkylsulfaten, insbesondere -methosulfaten, Benzyldimethylstearylammonium($C_1$-$C_4$)alkylsulfaten, insbesondere -methosulfaten, Stearamidopropyldimethyl (myristylacetat) ammonium($C_1$-$C_4$) alkylsulfaten, insbesondere -methosulfaten; und auch Mischungen davon; und vorzugsweise aus Dicetyldimethylammoniumhalogeniden, insbesondere -chloriden, oder Dicetyldimethylammonium($C_1$-$C_4$)alkylsulfaten, insbesondere -methosulfaten.

**4.** Zusammensetzung nach einem der vorhergehenden Ansprüche, umfassend das Tensid bzw. die Tenside der Formel

(Ia) in einer Gesamtmenge im Bereich von 0,1 Gew.% bis 10 Gew.%, besser 0,2 Gew.% bis 10 Gew.%, bevorzugt 0,5 Gew.% bis 5 Gew.%, bezogen auf das Gesamtgewicht der Zusammensetzung.

**5.** Zusammensetzung nach einem der vorhergehenden Ansprüche, wobei das kationische Tensid bzw. die kationischen Tenside, das/die sich von den kationischen Tensiden der Formel (Ia) unterscheidet/unterscheiden, ausgewählt ist/sind aus den kationischen Tensiden der Formel (Ib):

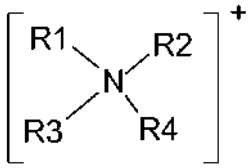 X (Ib)

worin:

- R1 für eine lineare oder verzweigte, gesättigte oder ungesättigte aliphatische Gruppe steht, die 8 bis 30 Kohlenstoffatome, vorzugsweise 12 bis 24 Kohlenstoffatome umfasst, wobei die aliphatische Gruppe möglicherweise ein oder mehrere Heteroatome umfasst, wie Sauerstoff, Stickstoff, Schwefel und/oder Halogene; vorzugsweise eine lineare oder verzweigte gesättigte aliphatische Gruppe, die 8 bis 30 Kohlenstoffatome, vorzugsweise 12 bis 24 Kohlenstoffatome umfasst, wobei die aliphatische Gruppe möglicherweise ein oder mehrere Heteroatome umfasst, wie Sauerstoff und/oder Stickstoff; noch besser eine gesättigte lineare $C_8$-$C_{30}$-, insbesondere $C_{12}$-$C_{24}$-Alkylgruppe oder eine ($C_{12}$-$C_{22}$)Alkylamido($C_2$-$C_6$)-alkylgruppe; vorzugsweise einen Behenyl-, Cetyl- oder Palmitylamidopropylrest,
- R2 bis R4, die identisch oder unterschiedlich sein können, für eine lineare oder verzweigte, gesättigte oder ungesättigte aliphatische Gruppe, die 1 bis 6 Kohlenstoffatome umfasst; insbesondere eine gesättigte lineare Alkylgruppe, die 1 bis 6 Kohlenstoffatome, besser noch 1 bis 3 Kohlenstoffatome umfasst; vorzugsweise eine Methylgruppe stehen,
- $X^-$ ein Anion ist, insbesondere ausgewählt aus Halogeniden wie Chlorid, Bromid und Iodid; Phosphaten, Acetaten, Lactaten, ($C_1$-$C_4$)Alkylsulfaten, ($C_1$-$C_4$)Alkyl- oder ($C_1$- $C_4$)Alkylaryl-sulfonaten.

**6.** Zusammensetzung nach einem der vorhergehenden Ansprüche, wobei das kationische Tensid bzw. die kationischen Tenside der Formel (Ib) ausgewählt ist/sind aus ($C_8$-$C_{30}$)Alkyltrimethylammoniumsalzen, und insbesondere Cetyltrimethylammoniumsalzen, Behenyltrimethylammoniumsalzen, Palmitylamidopropyltrimethylammoniumsalzen und Mischungen davon; insbesondere aus ($C_8$-$C_{30}$) Alkyltrimethylammoniumhalogeniden, insbesondere -chloriden, und insbesondere Cetyltrimethylammoniumhalogeniden, insbesondere -chlorid, Behenyltrimethylammoniumhalogeniden, insbesondere - chlorid, Palmitylamidopropyltrimethylammoniumhalogeniden, insbesondere -chlorid; oder ($C_8$-$C_{30}$) Alkyltrimethylammonium ($C_1$-$C_4$) alkylsulfaten, insbesondere -methosulfaten, und insbesondere Cetyltrimethylammonium($C_1$-$C_4$)alkylsulfaten, insbesondere -methosulfat, Behenyltrimethylammonium($C_1$-$C_4$)alkylsulfaten, insbesondere -methosulfat, Palmitylamidopropyltrimethylammonium($C_1$-$C_4$) alkylsulfaten, insbesondere -methosulfat; und auch Mischungen davon; und bevorzugt aus Cetyltrimethylammoniumhalogeniden, insbesondere -chloriden, oder Behenyltrimethylammoniumhalogeniden, insbesondere - chloriden, und Cetyltrimethylammonium($C_1$-$C_4$)-alkylsulfaten, insbesondere -methosulfaten, oder Behenyltrimethylammonium($C_1$-$C_4$) alkylsulfaten, insbesondere -methosulfaten.

**7.** Zusammensetzung nach einem der vorhergehenden Ansprüche, umfassend das Tensid bzw. die Tenside, das/die sich von den kationischen Tensiden der Formel (Ia) unterscheidet/unterscheiden, in einer Gesamtmenge im Bereich von 0,1 Gew.% bis 10 Gew.%, besser 0,2 Gew.% bis 10 Gew.%, bevorzugt 0,5 Gew.% bis 5 Gew.%, bezogen auf das Gesamtgewicht der Zusammensetzung.

**8.** Zusammensetzung nach Anspruch 5 oder 6, umfassend das Tensid bzw. die Tenside der Formel (Ib) in einer Gesamtmenge im Bereich von 0,1 Gew.% bis 10 Gew.%, besser 0,2 Gew.% bis 10 Gew.%, bevorzugt 0,5 Gew.% bis 5 Gew.%, bezogen auf das Gesamtgewicht der Zusammensetzung.

**9.** Zusammensetzung nach einem der vorhergehenden Ansprüche, wobei die Silikonmischung ein oder mehrere Polydialkylsiloxane, die Trialkylsilylendgruppen umfassen, der Formel (I) umfasst:

$$R'_3SiO(R'_2SiO)_pSiR'_3$$

worin:

- R', die identisch oder unterschiedlich sein können, ein einwertiger Rest auf Kohlenwasserstoffbasis mit 1 bis 18 Kohlenstoffatomen, vorzugsweise 1 bis 6 Kohlenstoffatomen, noch besser 1 bis 3 Kohlenstoffatomen, noch besser ein Methylrest ist, und
- p eine ganze Zahl im Bereich von 500 bis 2000, noch besser 1000 bis 2000 ist;

vorzugsweise mit einer Viskosität im Bereich von 40 000 bis 100 000 mPa·s bei 25 °C, vorzugsweise im Bereich von 40 000 bis 70 000 mPa·s bei 25 °C, noch besser 51 000 bis 70 000 mPa·s bei 25 °C.

**10.** Zusammensetzung nach einem der vorhergehenden Ansprüche, wobei die Silikonmischung ein oder mehrere Aminosilikone der Formel (II) umfasst:

$$XR_2Si(OSiAR)_n(OSiR_2)_mOSiR_2X$$

worin:

- R', die identisch oder unterschiedlich sein können, ein einwertiger Rest auf Kohlenwasserstoffbasis mit 1 bis 18 Kohlenstoffatomen, vorzugsweise 1 bis 6 Kohlenstoffatomen, noch besser 1 bis 3 Kohlenstoffatomen, noch besser ein Methylrest ist,
- X, die identisch oder unterschiedlich sein können, für R oder eine Hydroxylgruppe (OH) oder eine $C_1$-$C_6$-Alkoxygruppe steht; vorzugsweise X R ist, das bedeutet ein einwertiger Rest auf Kohlenwasserstoffbasis mit 1 bis 18 Kohlenstoffatomen, vorzugsweise 1 bis 6 Kohlenstoffatomen, besser 1 bis 3 Kohlenstoffatomen, noch besser ein Methylrest ist,
- A ein Aminorest der Formel $-R^1-[NR^2-R^3-]_xNR^2_2$ ist, oder die protonierte Form dieses Aminorestes, wobei

  - $R^1$ für einen $C_1$-$C_6$-Alkylenrest steht, vorzugsweise einen Rest $-CH_2CH_2CH_2-$ oder $-CH_2CH(CH_3)CH_2-$,
  - $R^2$, die identisch oder unterschiedlich sein können, ein Wasserstoffatom oder ein $C_1$-$C_4$-Alkylrest ist, vorzugsweise ein Wasserstoffatom,
  - $R^3$ ein $C_1$-$C_6$-Alkylenrest ist, vorzugsweise ein Rest $-CH_2CH_2-$,
  - x 0 oder 1 ist;

- m und n ganze Zahlen sind, so dass m+n im Bereich von 50 bis 1000, noch besser 50 bis 600 liegt;

vorzugsweise mit einer Viskosität bei 25 °C im Bereich von 1000 bis 15 000 mPa·s, vorzugsweise von 1500 bis 15 000 mPa·s, und/oder einer Aminzahl im Bereich von 2 bis 10 mg KOH pro Gramm Aminosilikon, vorzugsweise 3,5 bis 8 mg.

**11.** Zusammensetzung nach einem der vorhergehenden Ansprüche, umfassend (i) ein oder mehrere Polydialkylsiloxane, umfassend Trialkylsilylendgruppen, mit einer Viskosität bei 25 °C im Bereich von 40 000 bis 100 000 mPa·s, in einer Menge von 70 Gew.% bis 90 Gew.%, vorzugsweise 75 Gew.% bis 85 Gew.%, und (ii) ein oder mehrere Aminosilikone mit einer Viskosität bei 25 °C im Bereich von 1000 bis 15 000 mPa·s und einer Aminzahl im Bereich von 2 bis 10 mg KOH pro Gramm Aminosilikon, in einer Menge von 10 Gew.% bis 30 Gew.%, insbesondere 15 Gew.% bis 25 Gew.%, bezogen auf das Gesamtgewicht der Silikonmischung.

**12.** Zusammensetzung nach einem der vorhergehenden Ansprüche, wobei die Tensidmischung ein oder mehrere nichtionische Tenside umfasst, die ausgewählt ist/sind aus:

(i) (poly)oxyalkylenierten, insbesondere (poly)-ethoxylierten Fettalkoholen und insbesondere jenen der Formel: $R_3-(OCH_2CH_2)_cOH$, worin:

  - R3 für einen linearen oder verzweigten Alkyl- oder Alkenylrest steht, der 8 bis 40 Kohlenstoffatome und insbesondere 8 bis 30 Kohlenstoffatome umfasst, gegebenenfalls substituiert mit einer oder mehreren, insbesondere 1 bis 4 Hydroxylgruppen; und
  - c eine ganze Zahl im Bereich von 1 bis 200, insbesondere 2 bis 150 oder sogar 4 bis 50 und noch besser 8 bis 20 ist;

(ii) (poly) oxyalkylenierten ($C_8$-$C_{32}$) Alkylphenylethern, insbesondere umfassend 1 bis 200, besser noch 1 bis 30 Mol Ethylenoxid;
(iii) polyoxyalkylenierten Estern von $C_8$-$C_{32}$-Fettsäuren und Sorbitan, insbesondere polyoxyethylenierten Estern von $C_8$-$C_{32}$-Fettsäuren und Sorbitan, vorzugsweise mit 2 bis 40 Ethylenoxideinheiten, besser noch 2 bis 20 Ethylenoxid- (EO)-Einheiten; insbesondere polyoxyethylenierten Estern von $C_{10}$-$C_{24}$-Fettsäuren und Sorbitan, vorzugsweise mit 2 bis 40 Ethylenoxideinheiten, noch besser 2 bis 20 Ethylenoxid- (EO)-Einheiten; und
(iv) polyoxyethylenierten Estern von $C_8$-$C_{32}$-Fettsäuren, vorzugsweise mit 2 bis 150 Ethylenoxideinheiten; insbesondere polyoxyethylenierten Estern von $C_{10}$-$C_{24}$-Fettsäuren, umfassend insbesondere 2 bis 150 Ethylenoxid-(EO)-Einheiten.

**13.** Zusammensetzung nach einem der vorhergehenden Ansprüche, wobei die Öl-in-Wasser-Emulsion umfasst:

- die Tensidmischung in einer Gesamtmenge im Bereich von 5 Gew.% bis 15 Gew.%, insbesondere 8 Gew.% bis 15 Gew.%, noch besser 10 Gew.% bis 12 Gew.%, bezogen auf das Gesamtgewicht der Emulsion; und/oder
- das nicht-ionische Tensid bzw. die nicht-ionischen Tenside in einer Gesamtmenge im Bereich von 5 Gew.% bis 15 Gew.%, insbesondere 8 Gew.% bis 15 Gew.%, noch besser 10 Gew.% bis 12 Gew.%, bezogen auf das Gesamtgewicht der Emulsion;
- die Silikonmischung in einer Gesamtmenge im Bereich von 40 Gew.% bis 60 Gew.%, insbesondere 45 Gew.% bis 55 Gew.%, bezogen auf das Gesamtgewicht der Emulsion; und/oder
- das Polydialkylsiloxan bzw. die Polydialkylsiloxane, umfassend Trialkylsilylendgruppen, in einer Gesamtmenge im Bereich von 35 Gew.% bis 45 Gew.%, insbesondere 38 Gew.% bis 42 Gew.%, bezogen auf das Gesamtgewicht der Emulsion; und/oder
- das Aminosilikon bzw. die Aminosilikone in einer Gesamtmenge im Bereich von 5 Gew.% bis 15 Gew.%, insbesondere 8 Gew.% bis 12 Gew.%, bezogen auf das Gesamtgewicht der Emulsion; und/oder
- Wasser in einer Gesamtmenge im Bereich von 25 Gew.% bis 50 Gew.%, insbesondere 30 Gew.% bis 45 Gew.%, noch besser 35 Gew.% bis 42 Gew.%, bezogen auf das Gesamtgewicht der Emulsion.

**14.** Zusammensetzung nach einem der vorhergehenden Ansprüche, wobei die Öl-in-Wasser-Emulsion eine Partikelgröße D50 zwischen 100 und 300 nm, besser zwischen 150 und 250 nm oder sogar zwischen 160 und 200 nm hat.

**15.** Zusammensetzung nach einem der vorhergehenden Ansprüche, umfassend die Öl-in-Wasser-Emulsion in einer Gesamtmenge im Bereich von 0,1 Gew.% bis 10 Gew.%, besser 0,2 Gew.% bis 8 Gew.%, bevorzugt 0,5 Gew.% bis 6 Gew.%, bezogen auf das Gesamtgewicht der Zusammensetzung.

**16.** Zusammensetzung nach einem der vorhergehenden Ansprüche, auch umfassend einen oder mehrere Fettalkohole, insbesondere der Struktur R-OH, wobei R eine lineare oder verzweigte, gesättigte oder ungesättigte $C_8$-$C_{30}$-, vorzugsweise $C_{10}$-$C_{22}$-, noch besser $C_{12}$-$C_{24}$-Alkyl- oder Alkenylgruppen bezeichnet, R möglicherweise mit einer oder mehreren Hydroxylgruppen substituiert ist; vorzugsweise in einer Gesamtmenge im Bereich von 0,1 Gew.% bis 20 Gew.%, noch besser 0,5 Gew.% bis 15 Gew.%, vorzugsweise 1 Gew.% bis 10 Gew.%, bezogen auf das Gesamtgewicht der Zusammensetzung.

**17.** Kosmetisches Verfahren zur Behandlung, insbesondere zur Konditionierung von Keratinmaterialien und insbesondere Keratinfasern und insbesondere dem Haar, welches die Auftragung einer Zusammensetzung nach einem der vorhergehenden Ansprüche auf die Keratinmaterialien umfasst, gegebenenfalls gefolgt von einer Verweilzeit und/oder einem Spülschritt und/oder einem Trocknungsschritt.

## Revendications

**1.** Composition cosmétique, en particulier composition capillaire, comprenant :

(i) un ou plusieurs tensioactifs cationiques de formule (Ia) :

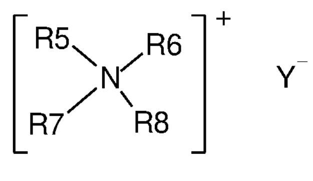

(Ia)

dans laquelle :

- R5 et R6, qui peuvent être identiques ou différents, représentent un groupe aliphatique, linéaire ou ramifié, saturé ou insaturé, comportant de 8 à 30 atomes de carbone, de préférence de 12 à 24 atomes de carbone, ledit groupe aliphatique pouvant comporter un ou plusieurs hétéroatomes tels que l'oxygène, l'azote, le soufre et/ou des halogènes ; ou un groupe aromatique tel qu'un groupe aryle ou alkylaryle, en particulier un groupe benzyle ;
- R7 et R8, qui peuvent être identiques ou différents, représentent un groupe aliphatique, linéaire ou ramifié, comportant de 1 à 6 atomes de carbone ;
- $Y^-$ est un anion;

(ii) un ou plusieurs tensioactifs cationiques différents des tensioactifs cationiques de formule (Ia), et
(iii) une émulsion huile-dans-eau ayant une taille de particules D50 inférieure à 350 nm, et comprenant :

- un mélange siliconé comprenant (i) un polydialkylsiloxane comprenant des groupes terminaux trialkylsilyle, ayant une viscosité à 25 °C allant de 40 000 à 100 000 mPa.s et (ii) une aminosilicone ayant une viscosité à 25 °C allant de 1 000 à 15 000 mPa.s et un indice d'amine allant de 2 à 10 mg de KOH par gramme d'aminosilicone ;
- un mélange tensioactif comprenant un ou plusieurs tensioactifs non ioniques, ledit mélange ayant un HLB allant de 10 à 16, et
- de l'eau,

la taille de particule, la viscosité, l'indice d'amine et les valeurs de HLB étant déterminés selon les procédés spécifiés dans la description.

2. Composition selon la revendication 1, comprenant un ou plusieurs tensioactifs cationiques de formule (Ia) dans laquelle :

- R5 représente un groupe alkyle linéaire saturé en C8-C30, en particulier C12-C24, ou un groupe alkyl(C12-C22)amidoalkyle(C2-C6) ; préférentiellement, un radical béhényle, cétyle, stéaryle ou stéaramidopropyle ; et/ou
- R6 représente un groupe alkyle linéaire saturé en C8-C30, en particulier C12-C24, ou un groupe alkyl(C12-C22)acétate ; préférentiellement, un radical benzyle, béhényle, cétyle, stéaryle ou myristylacétate ; et/ou
- R7 et R8, qui peuvent être identiques ou différents, représentent un groupe alkyle saturé linéaire comportant 1 à 6 atomes de carbone, en particulier de 1 à 3 atomes de carbone ; préférentiellement, un groupe méthyle.

3. Composition selon l'une ou l'autre des revendications précédentes, comprenant un ou plusieurs tensioactifs cationiques de formule (Ia) choisis parmi les sels de dialkyl(C8-C30)diméthylammonium, et en particulier les sels de distéaryldiméthylammonium, les sels de dicétyldiméthylammonium, les sels de benzyldiméthylstéarylammonium, les sels de stéaramidopropyldiméthyl-(myristylacétate)-ammonium et leurs mélanges ; plus particulièrement, parmi les halogénures, en particulier les chlorures, de dialkyl(C8-C30)diméthylammonium, et en particulier les halogénures, en particulier les chlorures, de distéaryldiméthylammonium, les halogénures, en particulier les chlorures, de dicétyldiméthylammonium, les halogénures, en particulier les chlorures, de benzyldiméthylstéarylammonium, les halogénures, en particulier les chlorures, de stéaramidopropyldiméthyl-(myristylacétate)-ammonium; ou bien parmi les alkyl(C1-C4)sulfates, en particulier les méthosulfates, de dialkyl(C8-C30)diméthylammonium, et en particulier les alkyl(C1-C4)sulfates, en particulier les méthosulfates, de distéaryldiméthylammonium, les alkyl(C1-C4)sulfates, en particulier les méthosulfates, de dicétyldiméthylammonium, les alkyl(C1-C4)sulfates, en particulier les méthosulfates, de benzyldiméthylstéarylammonium, les alkyl(C1-C4)sulfates, en particulier les méthosulfates, de stéaramidopropyldiméthyl-(myristylacétate)-ammonium ; ainsi que leurs mélanges ; et préférentiellement, parmi les halogénures, en particulier les chlorures, ou les alkyl(C1-C4)sulfates, en particulier les méthosulfates, de dicétyldiméthylammonium.

4. Composition selon l'une des revendications précédentes, comprenant le ou les tensioactifs de formule (Ia) en une quantité totale allant de 0,1 % à 10 % en poids, mieux de 0,2 % à 10 % en poids, préférentiellement de 0,5 % à 5 % en poids, par rapport au poids total de la composition.

5. Composition selon l'une des revendications précédentes, dans laquelle le ou les tensioactifs cationiques différents des tensioactifs cationiques de formule (Ia), sont choisis parmi les tensioactifs cationiques de formule (Ib) :

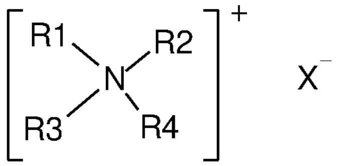
(Ib)

dans laquelle :

- $R^1$ représente un groupe aliphatique, linéaire ou ramifié, saturé ou insaturé, comportant de 8 à 30 atomes de carbone, de préférence de 12 à 24 atomes de carbone, ledit groupe aliphatique pouvant comporter un ou plusieurs hétéroatomes tels que l'oxygène, l'azote, le soufre et/ou les halogènes ; de préférence un groupe aliphatique saturé, linéaire ou ramifié, comportant de 8 à 30 atomes de carbone, de préférence de 12 à 24 atomes de carbone, ledit groupe aliphatique pouvant comporter un ou plusieurs hétéroatomes tels que l'oxygène et/ou l'azote ; encore mieux un groupe alkyle linéaire saturé en C8-C30, en particulier C12-C24, ou un groupe alkyl(C12-C22)amidoalkyle(C2-C6) ; préférentiellement, un radical béhényle, cétyle ou palmitylamidopropyle
- R2 à R4, qui peuvent être identiques ou différents, représentent un groupe aliphatique, linéaire ou ramifié, saturé ou insaturé, comportant de 1 à 6 atomes de carbone ; en particulier un groupe alkyle saturé linéaire comportant 1 à 6 atomes de carbone, mieux 1 à 3 atomes de carbone ; préférentiellement, un groupe méthyle
- $X^-$ est un anion, en particulier choisi parmi les halogénures comme chlorure, bromure et iodure ; les phosphates, les acétates, les lactates, les alkyl(C1-C4)sulfates, les alkyl(Cl-C4)- ou alkyl(C1-C4)aryl-sulfonates.

**6.** Composition selon la revendication précédente, dans laquelle le ou les tensioactifs cationiques de formule (Ib) sont choisis parmi les sels d'alkyl(C8-C30) triméthylammonium, et en particulier les sels de cétyltriméthylammonium, les sels de béhényltriméthylammonium, les sels de palmitylamidopropyltriméthylammonium et leurs mélanges ; plus particulièrement, parmi les halogénures, en particulier les chlorures, d'alkyl(C8-C30)triméthylammonium, et en particulier les halogénures, en particulier le chlorure, de cétyltriméthylammonium, les halogénures, en particulier le chlorure, de béhényltriméthylammonium, les halogénures, en particulier le chlorure, de palmitylamidopropyltriméthylammonium; ou les alkyl(Cl-C4)sulfates, en particulier les méthosulfates, d'alkyl(C8-C30)triméthylammonium, et en particulier les alkyl(C1-C4)sulfates, en particulier le méthosulfate, de cétyltriméthylammonium, les alkyl(C1-C4)sulfates, en particulier le méthosulfate, de béhényltriméthylammonium, les alkyl(C1-C4)sulfates, en particulier le méthosulfate, de palmitylamidopropyltriméthylammonium ; ainsi que leurs mélanges ; et préférentiellement, parmi les halogénures, en particulier les chlorures, de cétyltriméthylammonium ou de béhényltriméthylammonium, et les alkyl(C1-C4)sulfates, en particulier les méthosulfates, de cétyltriméthylammonium ou de béhényltriméthylammonium.

**7.** Composition selon l'une des revendications précédentes, comprenant le ou les tensioactifs différents des tensioactifs cationiques de formule (Ia) en une quantité totale allant de 0,1 % à 10 % en poids, mieux de 0,2 % à 10 % en poids, préférentiellement de 0,5 % à 5 % en poids, par rapport au poids total de la composition.

**8.** Composition selon l'une ou l'autre des revendications 5 et 6, comprenant le ou les tensioactifs de formule (Ib) en une quantité totale allant de 0,1 % à 10 % en poids, mieux de 0,2 % à 10 % en poids, préférentiellement de 0,5 % à 5 % en poids, par rapport au poids total de la composition.

**9.** Composition selon l'une des revendications précédentes, dans laquelle le mélange siliconé comprend un ou plusieurs polydialkylsiloxanes à groupes terminaux trialkylsilyle de formule (I) : R'3SiO(R'2SiO)pSiR'3 dans laquelle :

- R', qui peut être identique ou différent, est un radical hydrocarboné monovalent ayant 1 à 18 atomes de carbone, de préférence de 1 à 6 atomes de carbone, mieux de 1 à 3 atomes de carbone, encore mieux méthyle, et
- p est un entier allant de 500 à 2 000, mieux de 1 000 à 2 000 ;

de préférence ayant une viscosité allant de 40 000 à 100 000 mPa.s à 25 °C, de préférence allant de 40 000 à 70 000 mPa.s à 25 °C, mieux de 51 000 à 70 000 mPa.s à 25 °C.

**10.** Composition selon l'une des revendications précédentes, dans laquelle le mélange siliconé comprend une ou plusieurs aminosilcones de formule (II) :

$$XR_2Si(OSiAR)_n(OSiR_2)_mOSiR_2X$$

dans laquelle :

- R, qui peut être identique ou différent, est un radical hydrocarboné monovalent ayant 1 à 18 atomes de carbone, de préférence de 1 à 6 atomes de carbone, mieux de 1 à 3 atomes de carbone, encore mieux un radical méthyle,
- X, qui peut être identique ou différent, représente R ou un hydroxy (OH) ou un groupe alcoxy en C1-C6 ; de préférence X est R, c'est-à-dire un radical hydrocarboné monovalent ayant 1 à 18 atomes de carbone, de préférence de 1 à 6 atomes de carbone, mieux de 1 à 3 atomes de carbone, encore mieux méthyle,
- A est un radical amino de formule $-R^1-[NR^2-R^3-]_xNR^2_2$, ou la forme protonée de ce radical amino, avec
- $R^1$ représentant un radical alkylène en C1-C6, de préférence un radical $-CH_2CH_2CH_2-$ ou $-CH_2CH(CH_3)CH_2-$,
- $R^2$, qui peut être identique ou différent, étant un atome d'hydrogène ou un radical alkyle en C1-C4, de préférence un atome d'hydrogène,
- $R^3$ étant un radical alkylène en C1-C6, de préférence $- CH_2CH_2-$ ,
- x est 0 ou 1 ;
- m et n sont des entiers tels que m + n va de 50 à 1 000, mieux de 50 à 600 ;

de préférence ayant une viscosité à 25 °C allant de 1 000 à 15 000 mPa.s, de préférence de 1 500 à 15 000 mPa.s et/ou un indice d'amine allant de 2 à 10 mg de KOH par gramme d'aminosilicone ; de préférence de 3,5 à 8 mg.

**11.** Composition selon l'une des revendications précédentes, comprenant (i) un ou plusieurs polydialkylsiloxanes à groupes terminaux trialkylsilyle, ayant une viscosité à 25 °C allant de 40 000 à 100 000 mPa.s, en une quantité de 70 à 90 % en poids, de préférence de 75 à 85 % en poids, et (ii) une ou plusieurs aminosilicones ayant une viscosité à 25 °C allant de 1 000 à 15 000 mPa.s et un indice d'amine allant de 2 à 10 mg de KOH par gramme d'aminosilicone, en une quantité de 10 à 30 % en poids, en particulier de 15 à 25 % en poids, par rapport au poids total du mélange siliconé.

**12.** Composition selon l'une des revendications précédentes, dans laquelle le mélange tensioactif comprend un ou plusieurs tensioactifs non ioniques choisis parmi :

(i) les alcools gras (poly)oxyalkylénés, en particulier (poly)éthoxylés, et en particulier ceux de formule : $R_3-(OCH_2CH_2)_cOH$ dans laquelle :

- R3 représente un radical alkyle ou alcényle, linéaire ou ramifié, comportant de 8 à 40 atomes de carbone, en particulier 8 à 30 atomes de carbone, éventuellement substitué par un ou plusieurs, en particulier 1 à 4, groupes hydroxy ; et
- c est un entier allant de 1 à 200, en particulier de 2 à 150, voire de 4 à 50 et encore mieux de 8 à 20 ;

(ii) les (C8-C32)alkylphényle éthers (poly)oxyalkylénés, en particulier comprenant de 1 à 200, mieux de 1 à 30 moles d'oxyde d'éthylène ;
(iii) les esters polyoxyalkylénés d'acides gras en C8-C32 et du sorbitane, en particulier les esters polyoxyéthylénés d'acides gras en C8-C32 et du sorbitane, ayant de préférence de 2 à 40 motifs d'oxyde d'éthylène, mieux de 2 à 20 motifs d'oxyde d'éthylène (OE) ; en particulier les esters polyoxyéthylénés d'acides gras en C10-C24 et du sorbitane, ayant de préférence de 2 à 40 motifs d'oxyde d'éthylène, mieux de 2 à 20 motifs d'oxyde d'éthylène (OE) ; et
(iv) les esters polyoxyéthylénés d'acides gras en C8-C32, ayant de préférence de 2 à 150 motifs d'oxyde d'éthylène ; en particulier les esters polyoxyéthylénés d'acides gras en C10-C24, comprenant en particulier 2 à 150 motifs d'oxyde d'éthylène (OE).

**13.** Composition selon l'une des revendications précédentes, dans laquelle l'émulsion huile-dans-eau comprend :

- le mélange tensioactif en une quantité totale allant de 5 % à 15 % en poids, en particulier de 8 % à 15 % en poids, encore mieux de 10 % à 12 % en poids, par rapport au poids total de l'émulsion ; et/ou
- le ou les tensioactifs non ioniques en une quantité totale allant de 5 % à 15 % en poids, en particulier de 8 % à 15 % en poids, encore mieux de 10 % à 12 % en poids, par rapport au poids total de l'émulsion ;
- le mélange siliconé en une quantité totale allant de 40 % à 60 % en poids, en particulier de 45 % à 55 % en poids, par rapport au poids total de l'émulsion ; et/ou
- le ou les polydialkylsiloxanes à groupes terminaux trialkylsilyle en une quantité totale allant de 35 % à 45 % en poids, en particulier de 38 % à 42 % en poids, par rapport au poids total de l'émulsion ; et/ou

- la ou les aminosilcones en une quantité totale allant de 5 % à 15 % en poids, en particulier de 8 % à 12 % en poids, par rapport au poids total de l'émulsion ; et/ou
- de l'eau en une quantité totale allant de 25 % à 50 % en poids, en particulier de 30 % à 45 % en poids, encore mieux de 35 % à 42 % en poids, par rapport au poids total de l'émulsion.

**14.** Composition selon l'une des revendications précédentes, dans laquelle l'émulsion huile-dans-eau a une taille de particules D50 comprise entre 100 et 300 nm, mieux entre 150 et 250 nm, ou même entre 160 et 200 nm.

**15.** Composition selon l'une des revendications précédentes, comprenant l'émulsion huile-dans-eau en une quantité totale allant de 0,1 % à 10 % en poids, mieux de 0,2 % à 8 % en poids, préférentiellement de 0,5 % à 6 % en poids, par rapport au poids total de la composition.

**16.** Composition selon l'une des revendications précédentes, comprenant également un ou plusieurs alcools gras, en particulier de structure R-OH dans laquelle R désigne un groupe alkyle ou alcényle, linéaire ou ramifié, saturé ou insaturé, en C8-C30, de préférence en C10-C22, mieux en C12-C24, R pouvant être substitué par un ou plusieurs groupes hydroxy ; de préférence en une quantité totale allant de 0,1 % à 20 % en poids, mieux de 0,5 % à 15 % en poids, préférentiellement de 1 % à 10 % en poids, par rapport au poids total de la composition.

**17.** Procédé cosmétique pour le traitement, plus particulièrement pour le conditionnement, de matières kératiniques, et en particulier de fibres kératiniques et en particulier des cheveux, qui comprend l'application sur lesdites matières kératiniques d'une composition selon l'une des revendications précédentes, suivie éventuellement d'un temps de pose et/ou d'une étape de rinçage et/ou d'une étape de séchage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005213163 A
- JP 2000219612 A
- JP 2001139429 A
- US 2010015078 A1


### Non-patent literature cited in the description


- **GRIFFIN.** *J. Soc. Cosm. Chem.,* 1954, vol. 5 (4), 249-256
- **PUISIEUX ; SEILLER.** *Galenica 5: Les systèmes disperses [Galenics 5: Dispersed systems,* vol. I
- Agents de surface et emulsions [Surface agents and emulsions
- *Notions de HLB et de HLB critique [Notions of HLB and of critical HLB,* 153-194
- *Experimental determination of HLB,* 164-180
- **DAVIES JT.** A quantitative kinetic theory of emulsion type, I. Physical chemistry of the emulsifying agent. *Gas/Liquid and Liquid/Liquid Interface (Proceedings of the International Congress of Surface Activity),* 1957, 426-438
- **GODFREY.** Cationic emulsifiers in cosmetics. *J. Soc. Cosmetic Chemists,* 1966, vol. 17, 17-27